# EUROPEAN PATENT APPLICATION

(11) **EP 3 821 890 A1**
(43) Date of publication of application: **19.05.2021**
(21) Application number: 19209793.9
(22) Date of filing: 18.11.2019
(51) Int. Cl.: A61K 31/167, A61K 31/4045, A61K 31/437, A61K 31/4745, A61K 31/519, A61K 38/15, A61K 45/06, A61P 35/00

(54) **PHARMACEUTICAL COMPOSITIONS COMPRISING AN INHIBITOR OF A HISTON-DEACETYLASE (HDACI) AND AN AGONIST OF TOLL-LIKE-RECEPTOR 7 AND/OR 8 (TLR7 AND/OR TLR8) AND THEIR USE IN THE TREATMENT OF CANCER**

(71) Applicant: Strumberg, Dirk, 44869 Bochum (DE)
(72) Inventor: Díaz-Carballo, David, 45307 Essen (DE); Strumberg, Dirk, 44869 Bochum (DE)
(74) Representative: Grünecker Patent- und Rechtsanwälte PartG mbB

(57) **Abstract**

The invention relates to pharmaceutical compositions comprising at least one inhibitor of a histon-deacetylase (HDACi) and at least one agonist of Toll-like-receptor 7 and/or 8 (TLR7 and/or TLR8). The invention further relates to the aforementioned pharmaceutical compositions for use in a method of treating or preventing a disease. The invention further relates to the aforementioned pharmaceutical compositions for use in a method of treating or preventing cancer. The invention further relates to a method of treating or preventing cancer. The invention further relates to a kit of parts comprising at least one inhibitor of a histon-deacetylase (HDACi) and at least one agonist of Toll-like-receptor 7 and/or 8 (TLR7 and/or TLR8). The invention further relates to a cancer cell contacted with the pharmaceutical composition of the invention, wherein said cancer cell has an upregulated expression of one or more single-stranded RNA (ssRNA) genes, wherein the one or more ssRNA genes with upregulated expression are preferably endogenous retroviral (HERV) genes. The invention further relates to an *in vitro* method for contacting cancer cells with the pharmaceutical composition of the invention. The invention further relates to a population of cancer cells obtainable by the *in vitro* method of the invention.

## Description

The invention relates to pharmaceutical compositions comprising at least one inhibitor of a histon-deacetylase (HDACi) and at least one agonist of Toll-like-receptor 7 and/or 8 (TLR7 and/or TLR8). The invention further relates to the aforementioned pharmaceutical compositions for use in a method of treating or preventing a disease. The invention further relates to the aforementioned pharmaceutical compositions for use in a method of treating or preventing cancer. The invention further relates to a method of treating or preventing cancer. The invention further relates to a kit of parts comprising at least one inhibitor of a histon-deacetylase (HDACi) and at least one agonist of Toll-like-receptor 7 and/or 8 (TLR7 and/or TLR8). The invention further relates to a cancer cell contacted with the pharmaceutical composition of the invention, wherein said cancer cell has an upregulated expression of one or more single-stranded RNA (ssRNA) genes, wherein the one or more ssRNA genes with upregulated expression are preferably endogenous retroviral (HERV) genes. The invention further relates to an *in vitro* method for contacting cancer cells with the pharmaceutical composition of the invention. The invention further relates to a population of cancer cells obtainable by the *in vitro* method of the invention.

### Background of the invention

The medical need for new, more efficient anticancer treatments was the rationale to explore a novel therapeutic strategy by translating the "shock and kill" concept into anti-cancer therapy. "Shock and Kill" is a strategy intended to eliminate latent retroviruses from cell reservoirs. This comprises a two-step intervention: firstly latency-reversing agents are used to reactivate latent HIV (the "shock") and secondly the "kill" phase of those virus-expressing cells by the use of broadly neutralizing antibodies against viral structures. This idea was extrapolated to cancer treatments, taking into consideration that cancer cells basically express structural elements of endogenous retroviruses that could be enforcedly overexpressed by the use of some inhibitors of a histon-deacetylase (HDACis), which act as latency-reversing agents, as for example Vorinostat (SAHA) and Romidepsin. For the killing phase the inventors did not use antibodies against viral structures, as was done in the "kill phase" against HIV infection referred to above. Instead, the inventors took advantage of the innate immune system in the strategy described herein, as the TLR7 was stimulated using Vesatolimod (GS9620) or Imiquimod.

It is known that human endogenous retroviruses (HERVs) account for about 8% of the human genetic material as vestiges of ancestral germ-cell infections in which exoviruses were integrated into the host genome and transmitted in a Mendelian form to the progeny. HERVs retain all retroviral hallmarks, including the *gag, pol &. env* genes flanked by two regulatory, non-coding longterminal repeats (LTRs). In humans and other highly evolved anthropoid primates, HERVs have lost the capacity of horizontal transmission due to epigenetic repression or structural mutations. However, some of their genes still retain intact ORFs which could create transducing particles and therefore may be a part of bioactive quasispecies. Since HERV genes are normally repressed by epigenetic modifications, their reactivation/transactivation from "latency" in tumor development is a matter of current investigations.

As a model system, we chose SKOV3 ovarian cancer cells, since this cell type predominantly overexpresses HERV-V1 and HERV-V2 transcripts after HDACi exposure without affecting the overall protein levels. It appears that after the exposure to HDACi, HERV-V2 was more expressed than its truncated form, HERV-V1. TLR7 is expressed in this cell line as well as in other ovarian cancers. Moreover, some HERV elements have the ability to activate an innate immune response. Noteworthy, HERV-V1 and HERV-V2-derived protein is notably expressed in ovarian cancers but not in normal ovarian tissues. In addition to SKOV3 ovarian cancer cells, we also analyzed primary ovarian cancer cells isolated from ascites of heavily pretreated patients. Both drugs Vorinostat and Romidepsin are HDAC-inhibiting agents which are already in clinical use. The TLR7 agonists Imiquimod and Vesatolimod are also currently in clinical trials. Imiquimod is difficult to dissolve due to its hydrophobicity, whereas Vesatolimod has better dissolution profile in common vehicles which enables systemic use.

It is an aim of the invention to provide improved pharmaceutical compositions, uses and methods for treating or preventing cancer.

The combination of at least one inhibitor of a histon-deacetylase (HDACi) and at least one agonist of Toll-like-receptor 7 and/or 8 (TLR7 and/or TLR8) has not been previously considered as an anti-cancer treatment strategy. The inventors focused on ovarian carcinoma cells and breast cancer cells as model system. Nevertheless, this concept is plausibly applicable to other types of malignancies, preferably non-hematological malignancies.

### Summary of the invention

One aspect of the invention provides a pharmaceutical composition comprising at least one inhibitor of a histon-deacetylase (HDACi) and at least one agonist of Toll-like-receptor 7 and/or 8 (TLR7 and/or TLR8).

A further aspect of the invention provides a pharmaceutical composition comprising at least one inhibitor of a histon-deacetylase (HDACi) and at least one agonist of Toll-like-receptor 7 and/or 8 (TLR7 and/or TLR8) for use in a method of treating or preventing a disease, or for use in a method of therapy, or for use as a medicament, respectively.

A further aspect of the invention provides a pharmaceutical composition comprising at least one inhibitor of a histon-deacetylase (HDACi) and at least one agonist of Toll-like-receptor 7 and/or 8 (TLR7 and/or TLR8) for use in a method of treating or preventing cancer.

A further aspect of the invention provides a cancer cell contacted with the pharmaceutical composition of the invention, wherein said cancer cell has an upregulated expression of one or more single-stranded RNA (ssRNA) genes, wherein the one or more ssRNA genes with upregulated expression are preferably endogenous retroviral (HERV) genes.

A further aspect of the invention provides an *in vitro* method for contacting cancer cells with the pharmaceutical composition of the invention comprising the step of a) contacting cancer cells isolated from a cancer patient with the pharmaceutical composition of the invention.

A further aspect of the invention provides a population of cancer cells obtainable by the *in vitro* method of the invention.

### Brief description of the figures

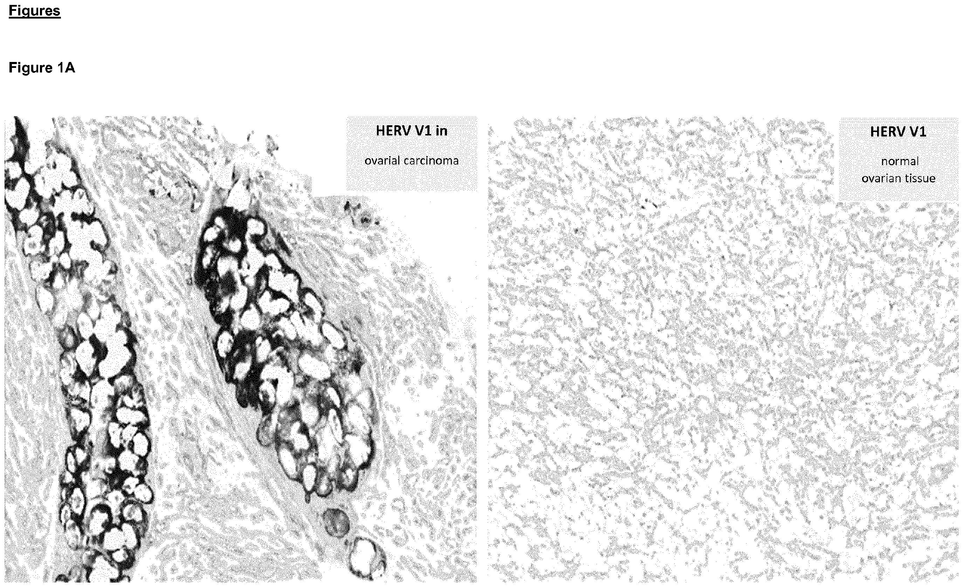

**Figure 1A** shows expression of HERV-V1 in ovarian cancers in comparison to normal ovarian tissue. Ovarian cancers regardless their histological classification express large quantities of HERV-V1 whereas normal tissue of this organ expresses very little quantities of this retroviral protein.

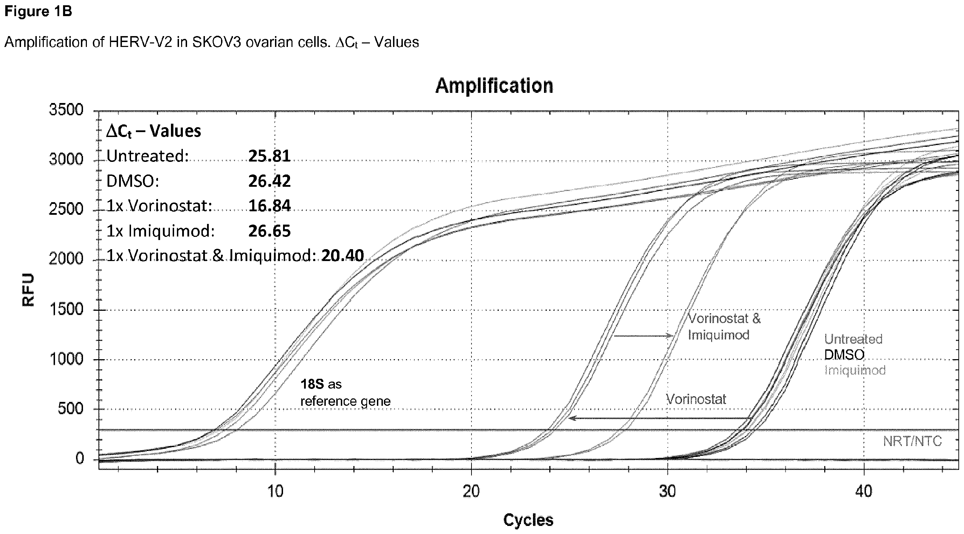

**Figure 1B** illustrates amplification of HERV-V2 transcription by Vorinostat in SKOV3 ovarian cancer cells via quatitative PCR (qPCR). 18S ribosomal RNA serves as standard. Additional treatment with Imiquimod slightly reduced amplification of HERV-V2 transcription. The term ΔCt reflects the difference (Δ) in cycle (C) thresholds (t). For standardized qPCR, the reference is usually the house-keeping gene 18S ribosomal RNA, which is always expressed in all cells in comparable quantity. Measurement of specific RNA transcripts like HERV-V1/V2 by qPCR is usually performed in relation to the standard 18S, more or less transcription products are expressed in + or - ΔCt.

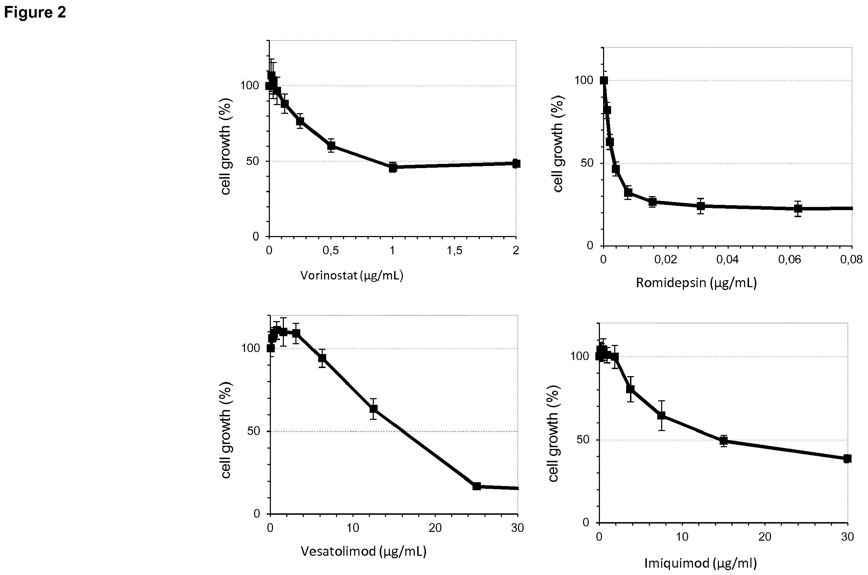

**Figure 2** shows the individual effects of the HDAC inhibitors Vorinostat and Romidepsin as well as of the agonists of TLR7 (Vesatolimod) and of TLR7/8 (Imiquimod), which were tested on the SKOV3 ovarian cell line. Cell growth was determined by the MTT proliferation assay and indicated in per cent. A cell growth of 50% indicates the respective IC₅₀ value.

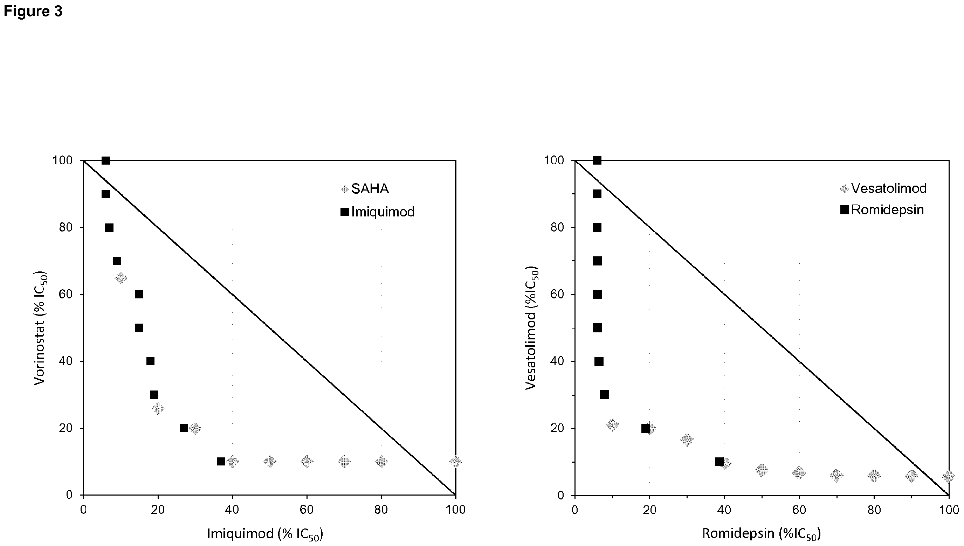

**Figure 3** shows isobologram-analyses obtained from the simultaneous exposure of the HDAC inhibitors Vorinostat or Romidepsin together with the TLR7/8 agonists Vesatolimod or Imiquimod in the SKOV3 ovarian cell line. The cross-combination Romidepsin-Imiquimod, Vorinostat-Vesatolimod showed comparable results.

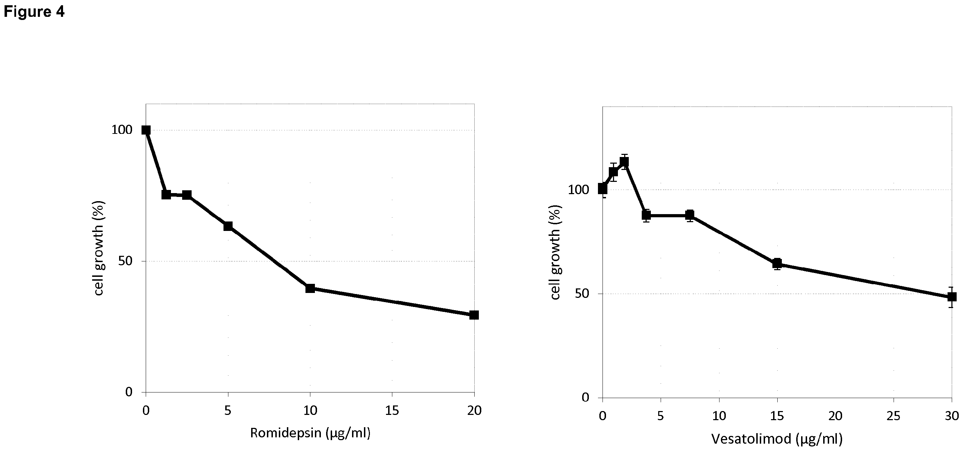

**Figure 4** shows the % cell growth of OvCa28 primary ovarian cancer cells isolated from pretreated patients. These primary ovarian cancer cells were treated individually with either the HDAC inhibitor Romidepsin or the TLR7 agonists Vesatolimod. Cell growth was determined by the MTT proliferation assay.

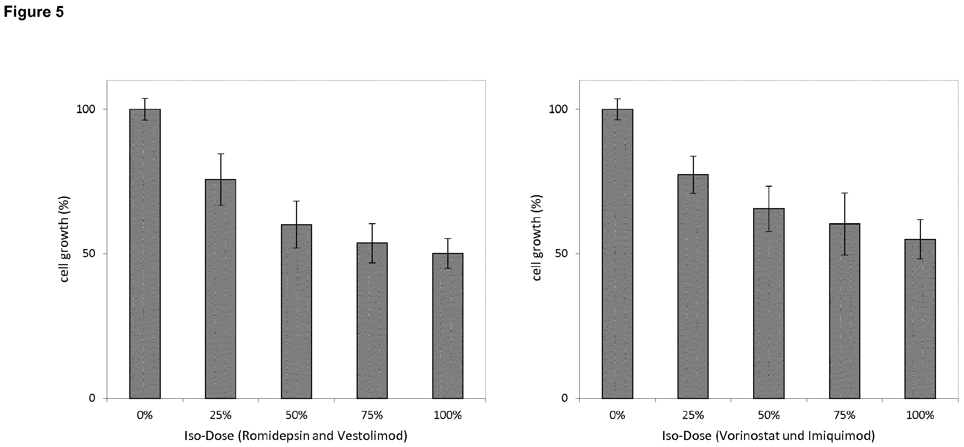

**Figure 5** shows the dose-response relationship obtained from the simultaneous incubation with Romidepsin and Vesatolimod as well as with Vorinostat und Imiquimod in OvCa28 primary ovarian cancer cells.

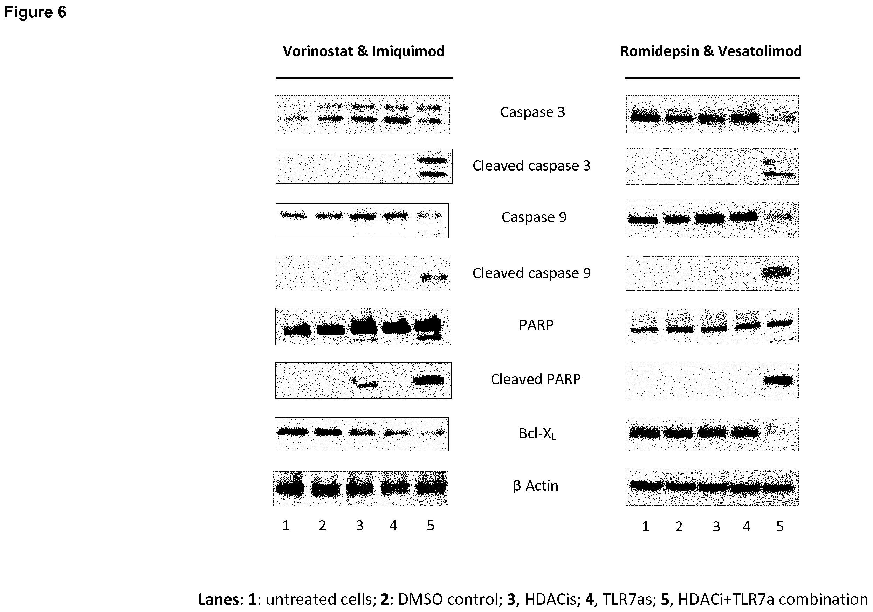

**Figure 6** shows the results regarding induction of apoptosis reflected by cleaved caspase 3 as well as cleaved PARP obtained only after the simultaneous exposure of SKOV3 ovarian carcinoma cells to HDACi and TLR7/8 agonists, as studied by the Western blot technique.

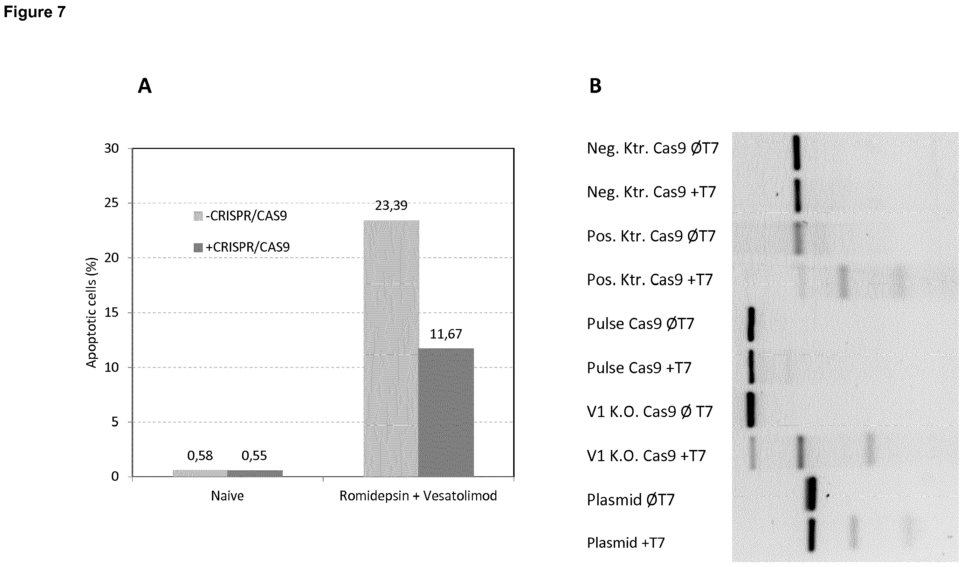

**Figure 7** shows the results in SKOV3 ovarian cancer cells after CRISPR/Cas9-induced silencing of HERV-V1/V2 genes. The left bars indicate negative-controls with or without gene solencing but without any drug treatment, showing almost no apoptotic cells.
   The right bars show cells treated with the HDAC-inhibitor romidepsin in combination with the selective TLR7 agonist vesatolimod. The light grey bar shows the positive-controls, i.e. SKOV cells without CRISPR/Cas9-induced silencing of HERV-V1/V2 genes.The expected high rate of apoptotic cells measured by annexin V reaches 24% in this experiment. In contrast, after silencing of HERV-V1/V2, the rate of apoptotic cells was reduced to 11% (dark grey bar). Remaining apoptosis in these cells is most likely due to enhanced transcription of other HERV-genes.
**Figure 8A** shows results with regular, non-malignant cells as a control (PBMCs). These cells did not show synergistic interaction after exposure to combinational HDACi and TLR7/8 agonists and rates of apoptosis < 9%.
**Figure 8B** shows results with regular, non-malignant cells as a control (PBMCs). These cells did not show enhanced transcription of HERV-V1/V2 upon incubation with HDACi.
**Figure 9** shows the dose-response relationship of various doses of HDAC inhibitors in SKOV3 cells, measured by qPCR. HDAC inhibitors induced the transcription of HERV-elements in SKOV3 cells. In particular, the expression of HERV-V1 and V2 was increased by incubation with HDACis, e.g. SAHA (Vorinostat) or Romidepsin.
**Figures 10A** and **10B** confirm synergistic anti-cancer activity of Romidepsin in combination with Vesatolimod also in-vivo by SKOV3- tumor ectopic xenograft ovarian carcinoma mouse model
**Figures 11A** and **11B** show the effects of a combination of HDACi and TLR7/8 agonists on inflammatory signaling. Components of the inflammasome downstream of MyD88 were not affected by incubation with the combination of HDACi and TLR7/8 agonists with regard to their expression levels.
**Figure 12** shows a Western blot analysis revealing the downregulation of elements of TLR7/8 signaling. Lanes 1 untreated, 2 vehicle control, 3 HDACis, 4 TLR7/8 agonists and 5 both drugs.
**Figure 13** shows Western blot analysis that demonstrates that major signal transduction pathways were found to be impaired by the combined exposure to HDACi and TLR7/8 agonist. In particular, Akt/PKB and Ras-MEK-ERK pathways were found interrupted after the simultaneous incubation with HDACi and TLR7/8 agonists as studied by Western blots. Lanes 1 untreated, 2 vehicle control, 3 HDACis, 4 TLR7/8 agonists and 5 both drugs.
**Figure 14** shows an isobologram demonstrating a synergistic effect of the combination of Romidepsin and Vesatolimod also in the breast cancer cell line MCF7. The breast cancer cell line MCF7 was incubated with a combination of HDACi (Romidepsin) and TLR7/8 agonist (Vesatolimod = GS9620). As demonstrated in the isobologram, the combination of Romidepsin and Vesatolimod also showed a synergistic, antiproliferative effect of the breast cancer cell line MCF7.

### Detailed description of the invention

### Definitions

The following provides definitions of various terms and expressions used in the application to describe the invention. Some terms (e.g. HDACi, agonist of TLR7 and/or TLR8, pharmaceutical composition, etc.) are recited identically in the description of different aspects or embodiments of the invention. It is to be understood that definitions of terms and expressions used in more than one aspect of the invention apply equally to any aspect of the invention described herein in which those terms and expressions appear. This applies equally regardless of where, i.e. which section, within the application such terms are defined or discussed.

In this application, the use of the singular (e.g. "a" or "the") may include the plural unless specifically stated otherwise. Further, the use of the term "including" as well as other grammatical forms such as "includes" and "included", is not limiting.

As used herein the term "comprising" has the broad standard meaning "including", "encompassing", or "containing". It includes the explicitly recited element(s), and also allows, but does not require, the presence of other or another element(s) not recited.

As used herein the term "about" when referring to a particular value, e.g. an endpoint or endpoints of a range, encompasses and discloses, in addition to the specifically recited value itself, a certain variation around the specifically recited value. Such a variation may for example arise from normal measurement variability, e.g. in the weighing or apportioning of various substances by methods known to the skilled person. The term "about" shall be understood as encompassing and disclosing a range of variability above and below an indicated specific value, said percentage values being relative to the specific recited value itself, as follows. The term "about" may encompass and disclose variability of ± 5.0%. The term "about" may encompass and disclose variability of from ± 4.9% to ± 0.1%. The term "about", in reference to the particular recited value, may encompass and disclose that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed. Unless stated otherwise, if the term "about" is recited before the first endpoint of a numerical range, this term refers to both the first endpoint of the range and the second endpoint of the range. For instance, a recited range of "about X to Y" should be read as "about X to about Y". The same applies for a recited range of weight ratios. For instance, a recited range of weight ratios of "about X:Y - A:B" should be read as a weight ratio of "(about X):(about Y) - (about A):(about B)".

Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from X to Y". Accordingly, the expressions of ranges as "X - Y", "of X to Y", "from X to Y", "of X - Y" and "from X - Y" are to be understood equivalently as meaning and disclosing a range encompassing the end value X, all values between X and Y, as well as the end value Y. In contrast, the designation of a range in the present application using the word "between" preceding two bracketing values X and Y, or two bracketing ratios, is to be understood as meaning and disclosing the specified range in which both endpoint values X and Y are excluded, but all values between the specified endpoint values X and Y are included.

As used herein, the term "pharmaceutical composition" encompasses and discloses any physical entity comprising or consisting of or consisting essentially of the respective recited substances. The physical form of the pharmaceutical composition is not restricted. For example, the term "pharmaceutical composition" encompasses and discloses a powder in which the recited substances are each present in powder form. As a further example, the term "pharmaceutical composition" also encompasses and discloses a liquid solution in which the recited substances are present in solubilized form. As a further example, the term "pharmaceutical composition" also encompasses and discloses an emulsion in which the recited substances are present. As a further example, the term "pharmaceutical composition" also encompasses and discloses a suspension in which the recited substances are present. In particular, the term "pharmaceutical composition" may be as defined herein below, and may be formulated for a desired route of administration. As used and disclosed herein, the term "pharmaceutical composition" also may be a pharmaceutical composition suitable for oral delivery, e.g. in the form of a tablet, including but not limited to an effervescent tablet or a multilayer tablet, a powder, e.g. in the form of a sachet, a hard capsule, a soft gel capsule, a syrup, a cachet, a troche, or a lozenge, a pastille, e.g. a gummy pastille, a salve, or a liquid preparation. The term "pharmaceutical composition" may also be a pharmaceutical composition suitable for delivery by a non-oral route, for example in the form of a suppository, a tablet, a hard capsule, a soft gel capsule, a cream, a gel, a patch or a liquid. Further dosage forms of the pharmaceutical composition as well as referred routes of administration are set out herein below.

As used herein, the term "inhibitor of a histon-deacetylase (HDACi)" encompasses and discloses substances having an inhibitory activity on one or more histon-deacetylases. As used herein, the term comprises but is not limited to the compounds vorinostat (SAHA, CAS-number: 149647-78-9, ATC-code: L01XX38), romidepsin (CAS number: 128517-07-7, ATC-code: L01XX39), and panobinostat (CAS-number: 404950-80-7, ATC code: L01XX42).

As used herein, the term "agonist of Toll-like-receptor 7 and/or 8 (TLR7 and/or TLR8)" encompasses and discloses substances exerting an agonistic activity on TLR7 and/or TLR8. As used herein, the term comprises but is not limited to the compounds vesatolimod (GS9620, CAS number: 1228585-88-3), imiquimod (CAS-number: 99011-02-6, ATC-code: D06BB10), and resiquimod (R 848, CAS-number: 144875-48-9).

As used herein, the term "pharmaceutically acceptable excipient" means and discloses any compounds that are used in the manufacture of tablets such as, but not limited to dispersing agents, surface active agents, inert diluents, granulating and disintegrating agents, binding agents, and lubricating agents.

As used herein, the term "cancer" means and discloses all known types of malignancies, including but not limited to non-hematological malignancies, such as ovarian cancer, breast cancer, pancreatic cancer and lung cancer.

As used herein, the term "chemoresistant-cancer" means and discloses a cancer that has not been any more (after extensive chemotherapy) or primary (without any chemotherapeutic pretreatment) shown to be susceptible or sensitive to chemotherapy.

As used herein, the term "cytotoxic drug" means and discloses drugs that have a lethal (i.e. "cytotoxic") effect on cells.

As used herein, the term "single-stranded RNA (ssRNA) gene" means and discloses genes encoding a single-stranded RNA molecule.

As used herein, the term "synergistic effect" or related terms such as "synergy" means and discloses the combined technical effect of two distinct entities of compounds, such as at least one inhibitor of a histon-deacetylase (HDACi) and at least one agonist of Toll-like-receptor 7 and/or 8 (TLR7 and/or TLR8), wherein said combined technical effect is not additive, but is greater than the sum of the individual technical effects these distinct entities of compounds have when used individually. A "synergistic effect" or "synergy", respectively, is usually demonstrated in the art by means of an isobologram, as known to the skilled person.

Although the application mentions discrete embodiments, it is to be understood that any embodiment, and the features therein, may be freely combined with any other embodiment and the features therein, even in the absence of an explicit statement to this effect. Such combinations of one embodiment with another, or of one or more features in any one embodiment with one or more features in any other embodiment, thus belong to the disclosure of the application as filed as understood by the skilled person.

### Description of the invention

Ovarian cancers regardless their histological classification expresses large quantities of HERV-V2 as well as its truncated form, HERV-V1, whereas normal tissues of this organ express none or only very little quantities of this retroviral RNA or its protein. This can be seen in Example 1 and **Figure 1****,** which show expression of HERV-V1 in ovarian cancers in comparison to normal ovarian tissue as negative control.

HERV-V1 and HERV-V2 are retroviral elements which are constitutively expressed in cancer cells at both RNA and protein levels. In particular, transcription of these HERV elements, which encode a retroviral envelope protein, can be enhanced by HDACi treatment, which was demonstrated for Vorinostat and Romidepsin, as can be seen in Example 2, **Table 1.**

It is known to use either HDACi or agonists of TLR7 and/or TLR8 individually as single compounds in therapy. HDAC inhibitors are currently in clinical use for treatment of hematologic malignancies as monotherapy. Both the HDAC inhibitors Vorinostat and Romidepsin are FDA-approved compounds. Agonists of TLR7 and/or TLR8, when used individually, are less cytotoxic and show IC₅₀-values of more than 15 µg/mL in *in vitro,* as demonstrated in Example 3, **Figure 2****.** For instance, Imiquimod, is a FDA-approved TLR7/8 agonist and registered for topical treatment of genital warts and basal cell carcinoma. In animal models, it was found to depict a bio-distribution with no considerable side effects. Vesatolimod is a TLR7 agonist and currently in clinical trials to treat Hepatitis B/C. Possible antitumoral effects of Vesatolimod have not been reported yet.

### Pharmaceutical compositions comprising at least one inhibitor of a histon-deacetylase (HDACi) and at least one agonist of Toll-like-receptor 7 and/or 8 (TLR7 and/or TLR8)

In addressing the problem of providing improved compositions for use in a method of treating cancer the inventors have surprisingly found that the combination of at least one inhibitor of a histon-deacetylase (HDACi) with at least one agonist of Toll-like-receptor 7 and/or 8 (TLR7 and/or TLR8) leads to a synergistic effect with regard to the induction of apoptosis that is not seen with healthy cells, such as PBMCs from healty donors. This advantageous effect is due to the combined administration of at least one inhibitor of a histon-deacetylase (HDACi) with at least one agonist of Toll-like-receptor 7 and/or 8 (TLR7 and/or TLR8).

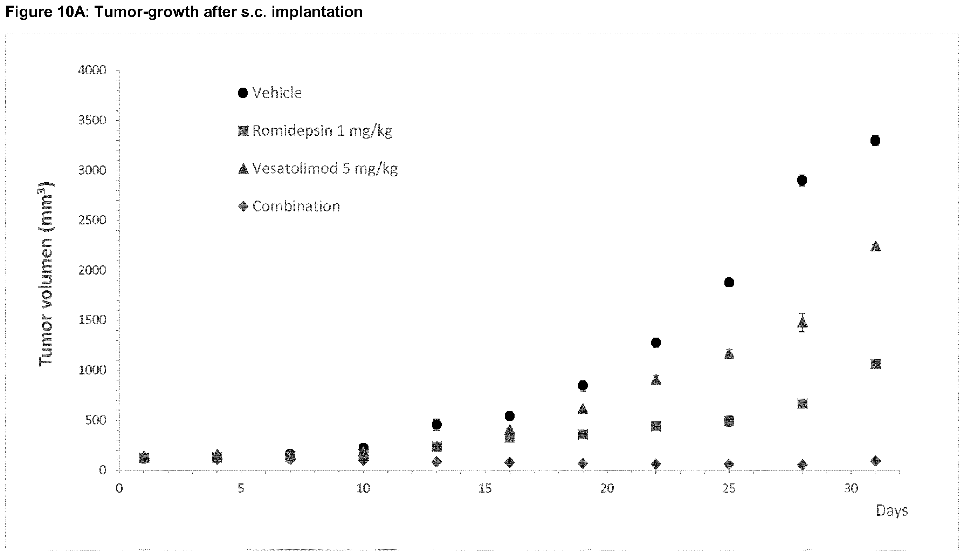

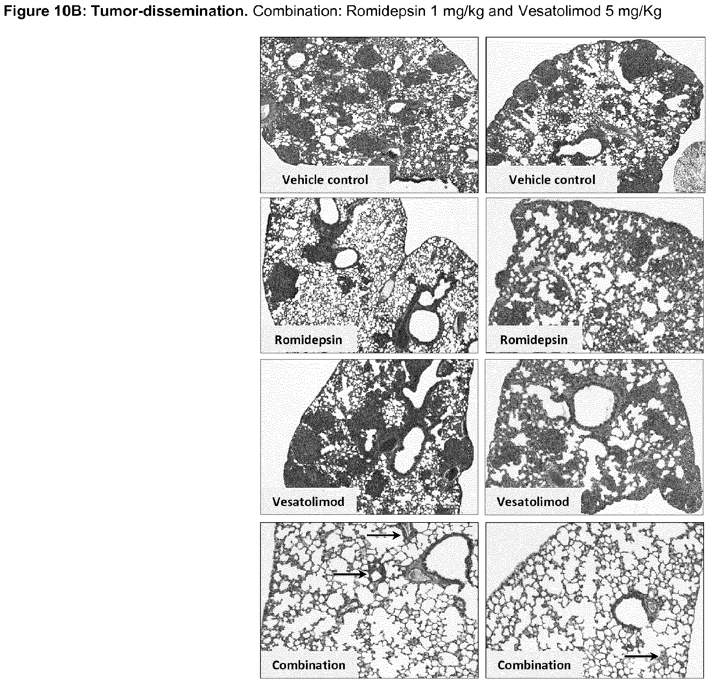

For instance, as described herein and shown in the later examples, the combination of HDACi with TLR7/8 agonists revealed a synergistic effect on cytotoxicity, reflected by specific induction of intrinsic apoptosis. This was observed in cell line models as well as in ovarian primary cancer cells from patients, but not in cells of healthy volunteers. Furthermore, this synergistic drug-effect was also confirmed in a mouse-model (**Figure 10** **A** and **B**).

While not being bound by theory, the inventors showed that HDACi greatly enforce the expression of HERV-V1 and V2 transcripts. Noteworthy, an exposure of cells to HDACi did not induce more amounts of HERV proteins in contrast to RNA transcripts. It is demonstrated herein that enhanced HERV-V1 and V2 gene transcription is the inducing event of the observed TLR7-mediated apoptosis, since specific gene silencing by CRISP/CAS9 resulted in significant reduction in cell death (see **Figure 7**). Remaining apoptosis after silencing of HERV-V2 is most likely related to other HERV-transcripts that are also induced by HDACi, but not silenced by this specific approach. Thus, these retroviral single-stranded RNA transcripts "trick" cancer cells to behave as if they were infected with a replicative virus. They interact with different elements of pattern recognition receptors with various downstream virus recognition pathways. In this case, TLR7/8 are the inducer of apoptosis, because without specific enhancement by TLR7/8 agonists major apoptosis does not take place.

In conclusion, the surprising finding of the inventors was that the combined administration of at least one inhibitor of a histon-deacetylase (HDACi) with at least one agonist of Toll-like-receptor 7 and/or 8 (TLR7 and/or TLR8) entails important therapeutic advantages in the treatment and/or prevention of cancer in-vitro as well in mouse-models, and also in cancer cells derived from heavily pretreated patients. Specifically, the combination of at least one inhibitor of an HDACi with at least one agonist of TLR7 and/or TLR8 leads to a synergistic effect with regard to the induction of apoptosis that is not seen in healthy cells, such as PBMCs from healty donors. Furthermore, this synergistic effect is dependent on a combination of certain drug-mechanisms representing different groups of drugs and not dependent on a single drug or a single drug-combination.

This has the ultimate effect of widening the scope of treating and/or preventing cancer and even extends to treating chemoresistant cancer, wherein the chemoresistant cancer can be resistant to cytotoxic drugs.

Accordingly, one aspect of the invention provides a pharmaceutical composition comprising, consisting essentially of, or consisting of at least one inhibitor of a histon-deacetylase (HDACi) and at least one agonist of Toll-like-receptor 7 and/or 8 (TLR7 and/or TLR8).

In a preferred embodiment of the invention, the pharmaceutical composition comprises one inhibitor of a histon-deacetylase (HDACi) and one agonist of Toll-like-receptor 7 and/or 8 (TLR7 and/or TLR8). However, the pharmaceutical composition of the invention can also comprise more than one HDACi and/or more than one agonist of TLR7 and/or TLR8 in any combination. For example, the pharmaceutical composition of the invention may comprise one, two, three or more HDACis and/or one, two, three or more agonists of TLR7 and/or TLR8 in any combination.

Preferably, the at least one inhibitor of a histon-deacetylase (HDACi) is selected from one or more of the group consisting of vorinostat (SAHA), romidepsin and panobinostat.

Preferably, the at least one agonist of Toll-like-receptor 7 and/or 8 (TLR7 and/or TLR8) is selected from one or more of the group consisting of vesatolimod (GS9620), imiquimod and resiquimod (R 848).

According to the invention, any combination of one or more HDACis as disclosed herein with one or more agonists of TLR7 and/or TLR8 as disclosed herein within the pharmaceutical composition is contemplated.

In a further embodiment, the invention also comprises co-adminsitration of any combination of one or more HDACis as disclosed herein with one or more agonists of TLR7 and/or TLR8 as disclosed herein to a subject within a given treatment regimen.

In one embodiment of the invention, the HDACi is romidepsin and the agonist of TLR7 and/or TLR8 is vesatolimod. In a further embodiment of the invention, the HDACi is vorinostat and the agonist of TLR7 and/or TLR8 is imiquimod.

Preferably, the pharmaceutical composition of the invention further comprises a pharmaceutically acceptable excipient.

The skilled person will understand that the "pharmaceutical composition" is intended to be used to treat or prevent certain pathological states as defined herein. The pharmaceutical composition may also be used to address physiological states in which an increased expression of single-stranded RNA (ssRNA) genes, such as endogenous retroviral (HERV) genes, in combination with an increased agonistic stimulation of Toll-like-receptor 7 and/or 8 (TLR7 and/or TLR8) is desireable. The term "pharmaceutical composition", as used herein, is not intended to be understood to be limited to a single physical entity, such as a single capsule or a single tablet, that has to comprise the at least one HDACi and the at least one agonist of TLR7/8. Rather, the term is to be understood to also comprise a combination of at least one HDACi and at least one agonist of TLR7/8, wherein the at least one agonist of TLR7/8 and the at least one HDACi are used within the same treatment regimen, but each are individually comprised in separate physical entities, such as capsules or tablets, for a non-simultaneous administration of the at least one HDACi and the at least one agonist of TLR7/8. In that sense, the invention also provides a combination of at least one inhibitor of a histone-deacetylase (HDACi) and at least one agonist of Toll-like-receptor 7 and/or 8 (TLR7 and/or TLR8). In this embodiment, the at least one HDACi and the at least one agonist of TLR7/8 can be comprised within different physical entities, for example a first physical entity and a second physical entity, such as a first tablet or capsule and a second tablet or capsule, which combined are also referred to as the "pharmaceutical composition" according to the invention.

Accordingly, the pharmaceutical composition of the invention can be in a corresponding medicinal form, and preferably comprises one or more pharmaceutically acceptable excipients.

Conversely, it is understood that establishing a certain amount of an inhibitor of a histon-deacetylase (HDACi) in the inventive pharmaceutical composition is sufficient to automatically establish a corresponding amount of an agonist of Toll-like-receptor 7 and/or 8 (TLR7 and/or TLR8) in the inventive pharmaceutical composition in order to achieve the above described synergistic technical effect.

As described above, the inventive pharmaceutical composition itself is intended for ultimate use in the treatment or prevention of a disease. More preferably, the inventive pharmaceutical composition itself is intended for ultimate use in the treatment or prevention of certain pathological conditions, preferably cancer. The pharmaceutical composition of the invention may also be used to address physiological states in which an increased expression of single-stranded RNA (ssRNA) genes, such as endogenous retroviral (HERV) genes, in combination with an increased agonistic stimulation of Toll-like-receptor 7 and/or 8 (TLR7 and/or TLR8) is desireable.

Accordingly, in a further embodiment of the invention, the pharmaceutical composition comprising at least one inhibitor of a histon-deacetylase (HDACi) and at least one agonist of Toll-like-receptor 7 and/or 8 (TLR7 and/or TLR8) is for use in a method of treating or preventing a disease, or is for use in a method of therapy, or is for use as a medicament, respectively.

In a further embodiment of the invention, the pharmaceutical composition comprising at least one inhibitor of a histon-deacetylase (HDACi) and at least one agonist of Toll-like-receptor 7 and/or 8 (TLR7 and/or TLR8) is for use in a method of treating or preventing cancer.

Preferably, the cancer is based on a non-hematological malignancy. Such non-hematological malignancy can be selected from the group consisting of ovarian cancer, breast cancer, pancreatic cancer and lung cancer.

In a preferred embodiment of the invention, the cancer is a chemo-resistant cancer. Preferably, the chemoresistant cancer is resistant to cytotoxic drugs, such as platinum compounds, taxanes, as well as etoposide.

In one aspect of the invention, the cancer is ovarian cancer, preferably chemo-resistant ovarian cancer, and more preferably chemo-resistant ovarian cancer that is resistant to cytotoxic drugs.

The pharmaceutical composition according to the invention can be administered parenterally, such as intravenously. Other modes of parenteral administration of the pharmaceutical composition contemplated by the invention are subcutaneous injection or infusion, or for administration in a bolus dose and/or continuous infusion.

In a further aspect of the invention, the pharmaceutical composition is administered orally.

In a further aspect of the invention, the at least one HDACi and the at least one agonist of TLR7/8 are not comprised within the same physical entity, but are comprised within separate physical entities, such as a first tablet or capsule and a second tablet or capsule, and are administered separately at defined time points within the same treatment regimen.

### a) Formulation of the inventive pharmaceutical composition for suitability for parenteral administration, preferably for intravenous administration

For parenteral administration, the pharmaceutical composition of the invention comprising at least one HDACi and at least one agonist of TLR7 and/or TLR8 may be formulated for injection or infusion, for example, intravenous, or subcutaneous injection or infusion, or for administration in a bolus dose and/or continuous infusion. Suspensions, solutions or emulsions in an oily or aqueous vehicle, optionally containing other agents such as suspending, stabilizing and/or dispersing agents such as those mentioned above, may be used.

The pharmaceutical composition of the invention comprising at least one HDACi and at least one agonist of TLR7 and/or TLR8 may be rendered especially suitable for parenteral administration by formulation with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules, crushable or otherwise, or in multi-dose containers containing a preservative. Compositions especially suitable for parenteral administration include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of the inventive pharmaceutical composition which is especially suitable for parenteral administration, the active ingredients are provided in dry (e.g. powder or granular) form for reconstitution with a suitable vehicle (e.g. sterile pyrogen free water) prior to parenteral administration of the reconstituted composition.

The inventive pharmaceutical composition may be prepared, packaged, or sold in the form of a sterile injectable aqueous or oily suspension or solution. This suspension or solution may be formulated according to the known art, and may comprise, in addition to the active ingredients, additional ingredients such as the dispersing agents, wetting agents, or suspending agents described herein. Such sterile injectable compositions may be prepared using a non toxic, parenterally acceptable diluent or solvent, such as water or 1,3-butanediol, for example. Other acceptable diluents and solvents include, but are not limited to, Ringer's solution, isotonic sodium chloride solution, and fixed oils such as synthetic mono- or diglycerides. Other usual parentally-administrable formulations include those that comprise the active ingredients in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

### b) Formulation of the inventive pharmaceutical composition for suitability for oral administration

A pharmaceutical composition of the invention suitable for oral administration may be prepared, packaged, or sold in the form of a discrete solid dose unit including a sachet (e.g. a powder in a sachet), a tablet, a hard or soft capsule, a cachet, a troche, or a lozenge, each containing a predetermined amount of at least one inhibitor of a histon-deacetylase (HDACi) and at least one agonist of Toll-like-receptor 7 and/or 8 (TLR7 and/or TLR8). Other formulations suitable for oral administration include a powdered or granular formulation, an aqueous or oily suspension, an aqueous or oily solution, or an emulsion. As used herein, an "oily" liquid comprises a carbon-containing liquid molecule that exhibits a less polar character than water.

It is especially preferred that the discrete solid dose unit of the inventive pharmaceutical composition is in the form of a powder, especially presented in the form of a sachet. As used herein, the term "sachet" refers to a sealed pouch containing the inventive composition. The pouch may be made of paper, wax paper, plasticized paper, or a combination of paper and foil. The material out of which the sachet is fashioned is preferably impervious to ambient moisture and other potential atmospheric contaminants so that, by sealing said sachet, the inventive composition in the form of a powder contained therein remains in a free flowing form until use.

A tablet comprising the at least one inhibitor of a histon-deacetylase (HDACi) and the at least one agonist of Toll-like-receptor 7 and/or 8 (TLR7 and/or TLR8) may, for example, be made by compressing or molding the HDACi and the agonist of TLR7 and/or TLR8, optionally with one or more additional ingredients, such as one or more pharmaceutically acceptable excipients. Compressed tablets may be prepared by compressing, in a suitable device, the HDACi and the agonist of TLR7 and/or TLR8 in a free flowing form such as a powder or granular preparation, optionally mixed with one or more of a binder, a lubricant, an excipient, a surface active agent, and a dispersing agent. Molded tablets may for example be made by molding, in a suitable device, a mixture of the HDACi and the agonist of TLR7 and/or TLR8, a pharmaceutically acceptable carrier, and at least sufficient liquid to moisten the mixture.

Pharmaceutically acceptable excipients used in the manufacture of tablets include, but are not limited to dispersing agents, surface active agents, inert diluents, granulating and disintegrating agents, binding agents, and lubricating agents. Known dispersing agents include, but are not limited to, potato starch and sodium starch glycolate. Known surface active agents include, but are not limited to, sodium lauryl sulfate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Tablets may be non-coated or they may be coated using known methods to achieve delayed disintegration in the gastrointestinal tract of a subject, thereby providing sustained release and absorption of the at least one HDACi and the at least one agonist of TLT7 and/or TLR8. Following ingestion, the composition of the invention will advantageously be released prior to passing to the small intestine, where primary HDACi and TLR7/TLR8-agonist resorption takes place. By way of example, a material such as glyceryl monostearate or glyceryl distearate may be used to coat tablets. Tablets may further comprise a sweetening agent, a flavoring agent, a coloring agent, a preservative, or some combination of these in order to provide pharmaceutically attractive and palatable preparation.

Hard capsules comprising the at least one HDACi and at least one agonist of TLR7 and/or TLR8 may be made using a physiologically degradable composition, such as gelatin or cellulose derivatives. Such hard capsules comprise the at least one HDACi and at least one agonist of TLR7 and/or TLR8, and may further comprise additional ingredients including, for example, an inert solid diluent such as calcium carbonate, calcium phosphate, or kaolin.

Soft gelatin capsules comprising comprising the at least one HDACi and at least one agonist of TLR7 and/or TLR8 may be made using a physiologically degradable composition, such as gelatin combined with a plasticizer (i.e. glycerol) as basic component of the soft gelatin shell. Soft gelatin capsules may contain a liquid or semisolid solution, suspension, or microemulsion preconcentrate. The soft capsule filling comprises the at least one HDACi and at least one agonist of TLR7 and/or TLR8, which may be mixed with water or an oil medium such as peanut oil, liquid paraffin, olive oil, soybean oil, sunflower oil, a lecithin such as for example soy lecithin or sunflower lecithin, medium chain triglycerides, polyglycerol oleate, beeswax, mono- and diglycerides of fatty acids, or combinations of any of the above.

Liquid formulations of the pharmaceutical composition of the invention that are especially suitable for oral administration may be prepared, packaged, and sold either in liquid form or in the form of a dry product intended for reconstitution with water or another suitable vehicle prior to ingestion.

Liquid suspensions may be prepared using conventional methods to achieve suspension of the active ingredients in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropyl methylcellulose. Known dispersing or wetting agents include, but are not limited to, naturally occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin and acacia. Known preservatives include, but are not limited to, methyl, ethyl, or n-propyl para-hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

Powdered and granular formulations of the pharmaceutical composition of the invention may be prepared using known methods. Such formulations may be administered directly to a subject, used, for example, to form sachet or tablets, to fill capsules, or to prepare an aqueous or oily suspension or solution by addition of an aqueous or oily vehicle thereto. Each of these formulations may further comprise one or more of dispersing or wetting agent, a suspending agent, and a preservative. Additional excipients, such as fillers and sweetening, flavoring, or coloring agents, may also be included in these formulations.

The pharmaceutical composition of the invention may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

Methods for impregnating or coating a material with a chemical composition are known in the art, and include, but are not limited to methods of depositing or binding a chemical composition onto a surface, methods of incorporating a chemical composition into the structure of a material during the synthesis of the material (e.g. such as with a physiologically degradable material), and methods of absorbing an aqueous or oily solution or suspension into an absorbent material, with or without subsequent drying.

### Therapeutic uses and methods

As described herein, the inventors have found that there is a synergistic therapeutic effect when at least one inhibitor of a histon-deacetylase (HDACi) is combined with at least one agonist of Toll-like-receptor 7 and/or 8 (TLR7 and/or TLR8) in treating or preventing cancer, in particular cancer that is based on a non-hematological malignancy.

Accordingly, a further aspect of the invention provides a combination of at least one inhibitor of a histon-deacetylase (HDACi) with at least one agonist of Toll-like-receptor 7 and/or 8 (TLR7 and/or TLR8) for use in a method of treating or preventing a disease, or for use in a method of therapy, or for use as a medicament, respectively. The at least one HDACi may be chosen from the types of HDACi set out above. The at least one agonist of TLR7 and/or TLR8 may be chosen from the types of agonists of TLR7 and/or TLR8 as defined hereinabove. As explained above, combining at least one HDACi and at least one agonist of TLR7 and/or TLR8 in the same regimen has a surprising synergistic effect, which can extend to treating even chemo-resistant cancer, such as cancer that is resistant to cytotoxic drugs.

As used herein, the term "combination", or the expression "in combination with" with reference to at least one inhibitor of a histon-deacetylase (HDACi) and at least one agonist of Toll-like-receptor 7 and/or 8 (TLR7 and/or TLR8), refers to the coupling of at least one HDACi and at least one agonist of TLR7 and/or TLR8 for the purpose of their coadministration to a subject within a given treatment regimen or within a given pharmaceutical composition. A given treatment regimen will typically comprise multiple, repeated coadministrations of at least one HDACi and at least one agonist of TLR7 and/or TLR8 over a certain time course, e.g. over 1 month, over 2 months, over 3 months, over 4 months or over 5 months or more. This time course and the duration of the treatment are usually determined by toxicity on the one hand and anti-tumor efficacy on the other hand. For instance, in patients with locally advanced or metastatic disease, this drug combination will be usually applied until disease progression or otherwise non-acceptable or non-manageble toxicity. The coadministration of at least one HDACi and at least one agonist of TLR7 and/or TLR8 is repeated over a predetermined length of time, and the sum of the instances of coadministration over this predetermined length of time constitutes the prophylactic or therapeutic regimen. Since for most drugs (HDACi and TLR7/8 agonists) oral formulations are available, an oral drug-combination might be preferred for clinical use. A reasonable starting point for a clinical application are published schedules and dosages of HDACi an dTLR7/8 agonists, that were already obtained in clinical studies:
**Vorinostat** is marketed only in the US under the name Zolinza by Merck for the treatment of cutaneous manifestations in patients with cutaneous T cell lymphoma (CTCL) when the disease persists, gets worse, or comes back during or after two systemic therapies. Vorinostat was the first histone deacetylase inhibitor approved by the U.S. Food and Drug Administration (FDA) for the treatment of CTCL on October 6, 2006. It also failed to demonstrate efficacy in treating acute myeloid leukemia in a phase II study. The recommended dosing schedule of Patients receiving oral vorinostat is 400 mg orally once daily.

**Panobinostat** (trade name Farydak) is a pan-HDACi by Novartis for the treatment of various cancers. On 23 February 2015 it received FDA accelerated approval for use in patients with multiple myeloma and on 28 August 2015 it was approved by the European Medicines Agency for the same use. Panobinostat is used in combination with the anti-cancer drug bortezomib and the corticoid dexamethasone for the treatment of multiple myeloma in adults who had received at least two previous treatments, including bortezomib and an immunomodulatory agent. Panobinostat is available for oral treatment (capsules of 10mg, 15mg, and 20mg). The recommended dosing regimen is 20 mg PO once every other day for 3 doses/week (on Days 1, 3, 5, 8, 10, and 12) of Weeks 1 and 2 of each 21-day cycle for 8 cycles.

**Romidepsin** (trade name Istodax), another histone deacetylase (HDAC) inhibitor, was approved by the FDA in November 2009 for the treatment of cutaneous T-cell lymphoma (CTCL) in patients who have received at least one prior systemic therapy and in May 2011 for the treatment of PTCL in patients who have received at least one prior therapy The drug is marked by Celgene Corporation since 2014. In 2012 the EMA refused approval for marketing authorisation in Europe. The drug is only approved for iv injection. The recommended dosing regimen is 14mg/m2 administered iv over a 4-hour period on days 1,8,15 of a 28-day cycle. The drug is also orally available, but orally formulations are so far only used in clinical trials.

**Imiquimod** (INN) is a prescription medication that acts as an immune response modifier that is used to treat genital warts, superficial basal cell carcinoma, and actinic keratosis. Scientists at 3M's pharmaceuticals division discovered the drug and 3M obtained the first FDA approval in 1997 under the brand Aldara. In 2004, 3M obtained FDA approval to market imiquimod as a treatment for superficial basal cell carcinoma. As of 2015, **imiquimod** is generic and is available worldwide under many brands. Imiquimod is an orally active interferon inducer with anti-tumour activity in experimental animals. In one study the tolerability, toxicity and biological effects of daily oral imiquimod administration were investigated in 21 patients with refractory cancer. Patients were treated with doses of 25 mg, 50 mg, 100 mg or 200 mg on a projected 112 day course.

**Vesatolimod** (GS-9620) is a potent and selective oral TLR7 agonist that directly activates plasmacytoid dendritic cells (pDCs). Vesatolimod is currently not approved and clinically developed by Gilead. Vesatolimod is orally available (tablets with 1mg, 2mg and 4 mg Vesatolimod used in clinical trials). The current clinical development focusses on hepatitis B and HIV.The dosing schedule in clinical trials is 8mg orally once every 14 days.

The coupling of at least one inhibitor of a histon-deacetylase (HDACi) and at least one agonist of Toll-like-receptor 7 and/or 8 (TLR7 and/or TLR8) meant by a "combination" of these two substances need not be physical (e.g. within a given pharmaceutical composition), nor need it imply simultaneity of administration. Reference to a "combination" of at least one HDACi and at least one agonist of TLR7 and/or TLR8 therefore encompasses and discloses multiple possibilities regarding the route and timing of administration of the respective substances. Encompassed and disclosed in the meaning of "combination" is for example the coupling of at least one HDACi and at least one agonist of TLR7 and/or TLR8 for simultaneous administration by the same route, simultaneous administration by different routes, chronologically staggered administration by the same route, or chronologically staggered administration by different routes. Any of the administration routes described hereinabove may be combined in any way, although it will generally be preferable that the route will be oral or parenteral, such as intraveneous. Especially preferable is simultaneous administration of at least one HDACi and at least one agonist of TLR7 and/or TLR8 by the oral route, e.g. when at least one HDACi and at least one agonist of TLR7 and/or TLR8 are present in a pharmaceutical composition, e.g. in one of the inventive pharmaceutical compositions set out herein, e.g. in the form of a tablet, hard capsule, soft gel capsule, or powder, e.g. in the form of a sachet, for ingestion.

Simultaneous administration of at least one HDACi and at least one agonist of TLR7 and/or TLR8 by the same route might for example take the form of at least one HDACi and at least one agonist of TLR7 and/or TLR8 being comprised in the same physical pharmaceutical composition, that pharmaceutical composition being administered to, e.g. ingested by, the subject so that the at least one HDACi and at least one agonist of TLR7 and/or TLR8 enter the body by the same route, preferably by the oral route, at the same time. Coupling of at least one HDACi and at least one agonist of TLR7 and/or TLR8 for staggered administration within a given coadministration is however also possible, and is also encompassed and disclosed in the term "combination". The order of administration of at least one HDACi and at least one agonist of TLR7 and/or TLR8 is not particularly important; the chronologically staggered administration of at least one HDACi and at least one agonist of TLR7 and/or TLR8 might encompass prior administration of at least one HDACi and subsequent administration of at least one agonist of TLR7 and/or TLR8, or prior administration of at least one agonist of TLR7 and/or TLR8 and subsequent administration of at least one HDACi. For example, staggered administration of at least one HDACi and at least one agonist of TLR7 and/or TLR8 by the same route may take the form of initial oral administration of at least one agonist of TLR7 and/or TLR8, followed by oral administration of at least one HDACi; coupling of at least one HDACi and at least one agonist of TLR7 and/or TLR8 in this way falls within the meaning of, and is disclosed by, "combination" as used herein. Conversely, staggered administration of at least one HDACi and at least one agonist of TLR7 and/or TLR8 by different routes might take the form of initial oral administration of at least one HDACi, followed by administration of at least one agonist of TLR7 and/or TLR8 by e.g. parenteral administration, or indeed, the other way round; coupling of at least one HDACi and at least one agonist of TLR7 and/or TLR8 in this way also falls within the meaning of, and is disclosed by, "combination" as used herein.

There are no particular restrictions on the duration of time between administration of at least one HDACi and at least one agonist of TLR7 and/or TLR8 in the event the coadministration is chronologically staggered, however it will generally be most effective and convenient when any interim between administering at least one HDACi and at least one agonist of TLR7 and/or TLR8 is brief, approaching simultaneity, e.g. on the order of minutes. In rare cases such an interim may extend up to about an hour or several hours between respective administrations. However, even in the event the at least one HDACi and the at least one agonist of TLR7 and/or TLR8 are administered in a chronologically staggered manner, whether by the same or different routes, the term "combination" means that a given coadministration of both at least one HDACi and at least one agonist of TLR7 and/or TLR8 within any regimen will have been completed, i.e. both substances will have been administered, by the time the respective next coadministration of the at least one HDACi and the at least one agonist of TLR7 and/or TLR8 (which itself may again be either simultaneous or chronologically staggered by the same or different routes) within the same regimen ensues.

A further aspect of the invention provides a method of treating or preventing a disease by administering a combination of at least one inhibitor of a histon-deacetylase (HDACi) and at least one agonist of Toll-like-receptor 7 and/or 8 (TLR7 and/or TLR8) to a patient in need thereof. The combination of at least one HDACi and at least one agonist of TLR7 and/or TLR8 can be within a pharmaceutical composition.

A further aspect of the invention provides a method of treating or preventing cancer by administering a combination of at least one inhibitor of a histon-deacetylase (HDACi) and at least one agonist of Toll-like-receptor 7 and/or 8 (TLR7 and/or TLR8) to a patient in need thereof. The combination of at least one HDACi and at least one agonist of TLR7 and/or TLR8 can be within a pharmaceutical composition.

As used herein, the term "treat" or grammatically related variants thereof such as "treatment", "treating" etc. means the amelioration, even temporarily, encompassing but not requiring the complete abolishment of a pathological state or disease, such as cancer.

As used herein, the term "prevent", and grammatically related terms such as "preventing", "prevention" etc. refer to scenarios in which the medical uses and methods of treatment described herein are applied to avert the possible, suspected or expected occurrence of a disease, wherein this disease preferably is cancer.

### Kits

A further aspect of the invention relates to a kit, e.g. a kit of parts, comprising at least one HDACi in a first container and/or tablet and at least one agonist of TLR7 and/or TLR8 in a second container and/or tablet.

The following provides several examples illustrating various embodiments of the present invention, and the technical effects and advantages which it achieves. It should be understood that the following examples are presented for illustrative purposes only, and do not limit the claimed invention. Indeed, the skilled person will readily be able to realize other embodiments within the spirit and scope of the claimed invention while achieving the describes technical advantages.

### Cancer cells, in vitro method and population of cancer cells

The invention further provides a cancer cell contacted with the pharmaceutical composition of the invention as described herein, wherein said cancer cell has an upregulated expression of one or more single-stranded RNA (ssRNA) genes following the contacting with the pharmaceutical composition. Preferably, the one or more ssRNA genes with upregulated expression are endogenous retroviral (HERV) genes.

In a preferred embodiment of the invention, the one or more HERV genes with upregulated expression comprise HERV-V2 and/or its truncated form, HERV-V1. In one preferred aspect of the invention, the one or more HERV genes with upregulated expression consist of HERV-V1 and/or HERV-V2.

Preferably, the cancer cell having an upregulated expression of one or more single-stranded RNA (ssRNA) genes, such as one or more HERV genes, e.g. HERV-V1 and/or HERV-V2, is an isolated cancer cell.

The invention further provides an *in vitro* method for contacting cancer cells with the pharmaceutical composition of the invention, as described herein, comprising the step of: a) contacting cancer cells isolated from a cancer patient with the pharmaceutical composition of the invention.

The invention further provides a population of cancer cells obtainable by the *in vitro* method of the invention, as described above.

### Examples

### General

In the broadest sense, the inventors have found that a combined administration of at least one inhibitor of a histon-deacetylase (HDACi) and at least one agonist of Toll-like-receptor 7 and/or 8 (TLR7 and/or TLR8) has a synergistic effect on cytotoxicity and the induction of apoptosis in cancer cells, which is absent in normal cells taken from healthy individuals.

The inventors have further found that HDACis greatly enhanced the expression of genes for HERV-V2 and the gene of the truncated form, HERV-V1, in a specific manner.

While not wanting to be bound by theory, the observed results suggest that at least enhanced HERV-V1 and V2 gene transcription is the inducing event of the observed TLR7/8-mediated apoptosis, since gene silencing of HERV-V1 and HERV-V2 genes resulted in significant reduction in cell death (see **Figure 7**). Thus, these retroviral elements "trick" cancer cells to behave as if they were infected with a replicative virus. They interact with different elements of pattern recognition receptors with various downstream virus recognition pathways. TLR7/8 are the inducer of apoptosis, because without specific enhancement by TLR7/8 agonists major apoptosis does not take place.

### EXAMPLE 1

### Expression of HERV-V1 in ovarian cancer cells

Ovarian cancers regardless their histological classification express large quantities of HERV, especially HERV-V1 and/or HERV-V2, whereas normal tissues of this organ express very little quantities of these retroviral proteins. This was demonstrated by immunocytochemistry and immunohistochemistry as shown in **Figure 1A****,** where the expression of HERV-V1 in ovarian cancers is shown in comparison to normal ovarian tissue.

In brief, sections of ovarian cancer tissues and control sections of normal ovarian tissue were analyzed by immunohistochemistry using antibodies specific for certain HERV-proteins including HERV-V1 as shown in **Figure 1A****.** Furthermore, a commercially available collection of regular tissue sections were also analyzed. We could confirm lacking or only sparce expression of HERV-elements in the control sections of normal ovarian tissue we analyzed (data not shown).

In addition, we performed quantitative PCR (qPCR) on SKOV3 ovarian cancer cells as shown in **Figure 1B**. These results demonstrate that HERV-V2 expression levels in SKOV3 ovarian cancer cells are increased after incubation with Vorinostat (a HDACi) and, less so, after incubation with a combination of Vorinostat (a HDACi) and Imiquimod (an agonist of TLR7/8).

The results presented in this experiment show that ovarian cancers express large quantities of HERV, especially HERV-V1 and HERV-V2, whereas normal ovarian tissues express only minor quantities of these retroviral proteins. Incubation with a HDACi, such as Vorinostat; substantially increased expression of HERV-V2 in SKOV3 ovarian cancer cells even further.

### EXAMPLE 2

### Expression of various HERV genes in ovarian cancer cells

HERV-V1 and HERV-V2 are retroviral elements which are constitutively expressed in cancer cells at both RNA and protein levels. In particular, transcription of these HERV elements, which encode a retroviral envelope protein, can be enhanced by HDACi treatment, which we demonstrated for Vorinostat and Romidepsin, as shown in this example (see Table 1 below).

In particular, the SKOV3^{WT} ovarian carcinoma cell line was screened for HERV expression using quantitative PCT (qPCR). The HERV-V1 and V2 transcripts were found predominantly expressed after exposure to HDAC inhibitors Vorinostat or Romidepsin. Gene expression was considered enforced when more than 3 PCR-cycles of difference (Untreated - Treated) was calculated (n≥3).

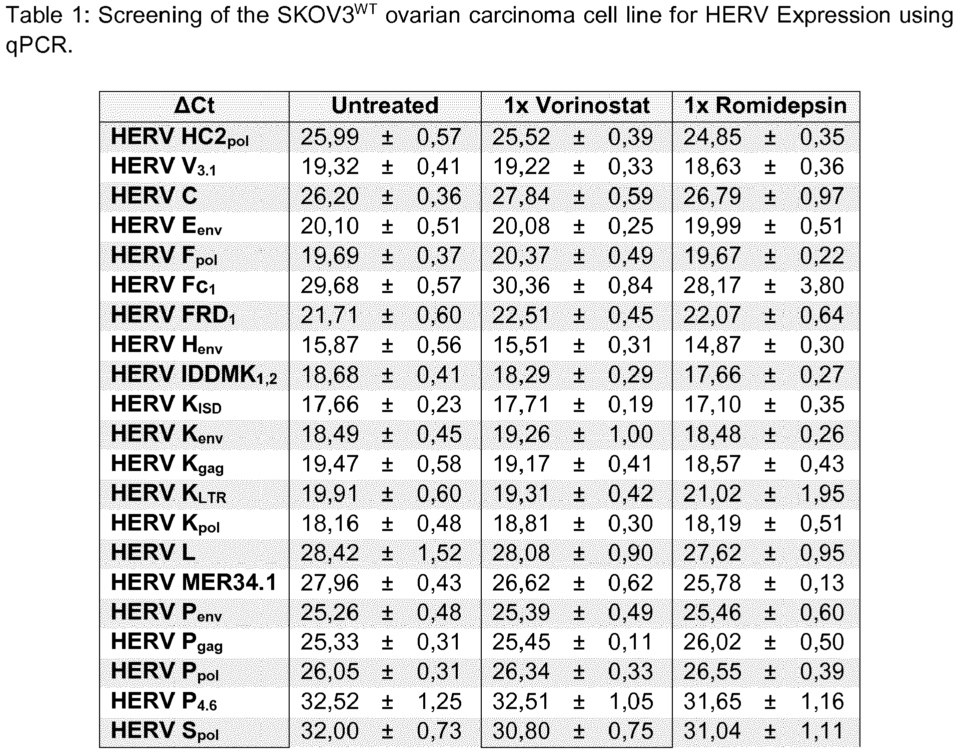

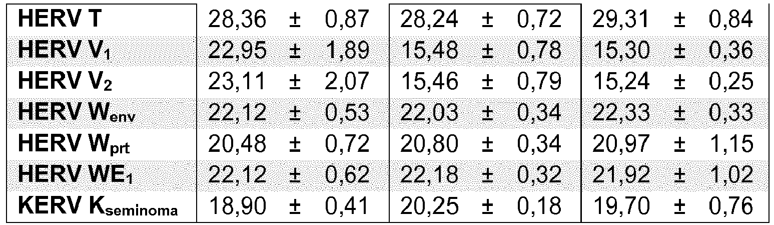

**Figure 1B** further illustrates amplification of HERV-V2 transcription by Vorinostat in SKOV3 ovarian cancer cells via quatitative PCR (qPCR). 18S ribosomal RNA serves as standard.The term ΔCt reflects the difference (Δ) in cycle (C) thresholds (t). For standardized qPCR, the reference is usually the house-keeping gene 18S ribosomal RNA, which is always expressed in all cells in comparable quantity. Measurement of specific RNA transcripts like HERV-V1/V2 by qPCR is usually performed in relation to the standard 18S, more or less transcription products are expressed in + or- ΔCt.

In view of the above, these results demonstrate that HERV-V1 and V2 transcripts were predominantly expressed in the SKOV3^{WT} ovarian carcinoma cell line after exposure to the HDAC inhibitors Vorinostat or Romidepsin.

### EXAMPLE 3

### Individual effects of HDACi and agonists of TLR7 and/or TLR8 on ovarian cancer cells

This example compares the individual effects of HDACis and agonsist of TLR7/8 on ovarian cancer cells. Both the HDAC inhibitors Vorinostat and Romidepsin are FDA-approved compounds. Agonists of TLR7 and/or TLR8 are less cytotoxic than HDACis and showed IC₅₀- values of more than 15 µg/mL in *in vitro,* as demonstrated in this example and shown in **Figure 2****.**

In brief, the individual effects of the HDAC inhibitors Vorinostat and Romidepsin as well as of the TLR7/8 agonists Vesatolimod and Imiquimod were tested on the SKOV3 ovarian cancer cell line. Cell growth was determined by the MTT proliferation assay and indicated in per cent, as shown in **Figure 2****.** A cell growth of 50% indicates the respective IC₅₀ value.

### EXAMPLE 4

### Synergistic effects of HDACi and agonists of TLR7 and/or TLR8 on ovarian cancer cells

The synergistic antiproliferative effect of a combination of inhibitors of histone-deacetylase (HDACi) and TLR7 and/or TLR8 agonists were first studied in the SKOV3 ovarian cancer cell line. The simultaneous use of HDACi and TLR7/TLR8 agonists revealed a significant synergistic interaction between both drug classes (i.e. HDACi and TLR7/TLR8 agonists), as depicted in the isbologram-analyses shown in **Figure 3****.**

The endpoint used in **Figure 3** is inhibition of proliferation by 50% (IC₅₀). Specifically, all dots in Figure 3 reflect a combination of HDACi plus TLR7/8 agonist, which resulted in 50% growth inhibition. All dots on the diagonal reflect additive activity (or lacking interaction), for instance 100% IC₅₀ of drug A und 0% IC₅₀ of drug B or vice versa. Dots above the diagonal would reflect antagonistic interactions, which was not a result of this experiment. However, all dots in experiments carried out for the results shown in **Figure 3** below the diagonal strongly suggest synergistic antiproliferative interactions between all analyzed HDACis and agonists of TLR7/TLR8. For instance, 20% IC₅₀ of Vorinostat plus 20% IC₅₀ of imiquimod resulted in the same antiproliferative activity as 100% IC₅₀ of each drug alone. Even stronger synergism was shown for the combination of vesatolimod and romidepsin. Thus, only low doses of both drugs resulted in enhanced antiproliferative activity when used in combination.

### EXAMPLE 5

### Synergistic effects of HDACi and agonists of TLR7 and/or TLR8 in ovarian cancer cells from pretreated patients

Further analyses in primary ovarian cancer cells from patients were performed. A total of 10 ovarian cancer patients were selected, all of them pretreated with chemotherapy. Cancer cells were isolated from ascites and investigated. Although all cells were resistant against both drug classes (HDACi and TLR7/8 agonists, respectively) as single drugs (see **Figure 4**), they showed the same synergistic interaction in combinational treatment, even using low doses of their original IC₅₀-values, depicted in **Figure 5****.**

OvCa28 primary ovarian cancer cells were isolated from a pretreated patient (patient number 28). These primary ovarian cancer cells were treated individually with either the HDAC inhibitor Romidepsin or the TLR7 agonist Vesatolimod as shown in **Figure 4**. Cell growth was determined by the MTT proliferation assay. The resulting IC₅₀ values were IC₅₀ = 7.5 µg/mL for Romidepsin and IC₅₀ = 30 mg/mL for Vesatolimod. For reference, in the SKOV3 cell line the IC₅₀-values for these drugs were 0.004 µg/mL for Romidepsin and 12.5 µg/mL for Vesatolimod, respectively. The results presented in this example show that primary ovarian cancer cells were more resistant against both drug classes - i.e. in the order of 188-fold for Romidepsin and 2-fold for Vesatolimod - as compared to the reference cell line SKOV3 ovarian carcinoma.

However, as far as any synergistic effects are concerned, it was found that the combination of HDACi with TLR7/8 agonists on OvCa28 primary ovarian cancer cells showed synergistic interactions that were comparable to the SKOV3 ovarian carcinoma cell line (see **Figure 5**).

**Figure 5** shows a dose-response diagram using OvCa28 primary ovarian cancer cells that were highly resistant against standard chemotherapy, including platinum compounds. These cells received simultaneous incubation with Romidepsin and Vesatolimod as well as with Vorinostat und Imiquimod. The IC₅₀-values employed for both drugs were those obtained from SKOV3 cells. Thus, in comparison to SKOV3 ovarian cells, for Romidepsin their IC₅₀ were 188-fold less and for Vesatolimod 2-fold less in OvCa28 primary ovarian cancer cells as their respective IC₅₀-values (for reference, see **Figure 4**).

### EXAMPLE 6

### Synergistic effects of HDACi and agonists of TLR7 and/or TLR8 on inducing apoptosis

The synergistic cytotoxic interaction for the combination of HDACi and TLR7/8 agonists induced selective intrinsic apoptosis, which was not (or less) seen for the single drug exposure as depicted in **Figure 6****.**

In particular, the results shown in **Figure 6** demonstrate major apoptosis observed only after the simultaneous exposure of SKOV3 ovarian carcinoma cells to HDACi and TLR7/8 agonists, as studied by the Western blot technique (**see** **Figure 6****, lane 5**).

In brief, SKOV3 cells were either untreated as negative controls (lanes 1 untreated, 2 vehicle control), or treated with the on top specified HDACi alone (lane 3), or treated with the on top specified TLR7/8 agonist alone (lane 4), or treated with a combantion of both drugs (lane 5) The specific doses used for all experiments were the respective IC₅₀-values obtained in the SKOV3 cell line. Intrinsic apoptosis was always found more detectable for the combined exposure to both drug classes (representative pictures).

The analysis of apoptotic pathways in this experiment revealed important clues for the mechanisms activated by the combination of HDACi and TLR7/8 agonists. Apoptotic cell death was reflected by a proteolytic cleavage of caspases 9 and 3 as well as PARP (poly ADP ribose polymerase). Caspase 9 cleavage reflects intrinsic apoptosis processes with downstream caspase 3 cleavage (see **Figure 6**). Importantly, PARP detects and initiates an immediate repair response to single-strand DNA breaks which is also a hallmark of apoptosis. PARP intramolecular digestion leads to an inactivation of this pivotal enzyme and cells undergo death by accumulating DNA-damage which in turn leads to an instable genome.

### EXAMPLE 7

### Abrogation of HERV-V1/V2 transcription by the CRISPR-CAS9 system significantly reduces apoptotic cell death by HDACi (Romidepsin) in combination with agonists of TLR7/8 (Vesatolimod)

**Figure 7A** and **7B** shows the results in SKOV ovarian cancer cells after CRISPR/Cas9-induced silencing of HERV-V1/V2 genes. The left bars in **Figure 7A** indicate negative-controls with or without gene silencing but without any drug treatment, showing almost no apoptotic cells.

The right bars in **Figure 7A** show cells treated with the HDAC-inhibitor romidepsin in combination with the selective TLR7 agonist vesatolimod. The light grey bar shows the positive-controls, i.e. SKOV cells without CRISPR/Cas9-induced silencing of HERV-V1/V2 genes.The expected high rate of apoptotic cells measured by annexin V reaches 24% of the total number of cells in this experiment. In contrast, after silencing of HERV-V1/V2, the rate of apoptotic cells was reduced to 11% (dark grey bar). Remaining apoptosis in these cells is most likely due to enhanced transcription of other HERV-genes.

Significant reduction of apoptotic cells after silencing of HERV-V1/V2 genes plausibly demonstrates, that HDACi-induced HERV transcription serves as an inducer or amplifier of TLR7/8 mediated apoptosis that is dramatically enhanced by additional application of specific agonists of TLR7/8.

**Figure 7B** shows the ablation of HERV-V1 using the CRISPR/Cas9 system. This was confirmed using the endonuclease T7 which recognizes a DNA modification in comparison with the wildtype.

The CRISPR/Cas9 (clustered regularly interspaced short palindromic repeats-CRISPR associated) system was used to generate stable double-strand breaks in DNA to knock-out HERV genes. We use the Alt-R™ CRISPR/Cas9 System (IDT Technologies, Iowa, USA), which uses the genetically modified S.p. Cas9 Nuclease 3NLS combined with the designed Alt-R CRISPR crRNA 35-36 nt RNA oligo containing the 20 nt target-specific for HERVs and the Alt-R CRISPR tracrRNA (conserved 67 nt RNA sequence that is required for complexing to the crRNA). The use of truncated gRNA (truncated within the crRNA-derived sequence) improves the CRISPR system in reducing off-target mutations.

Several CRISPR/Cas9 studies have reported that the simultaneous use of dual gRNAs results in a much better gene editing efficiency and specificity when compared with the use of a single gRNA. Taking this experience into consideration, we designed at least two different gRNAs for each HERV target (score > 50). Two gRNAs were selected for gene disruption:A:TATATATCATAAAGAAAACTCGG and B: GATCAGCCCATATGGACAACAGG. The guided sequences were aligned to the human genome to look for sites with similarity to other HERVs and tested for possible off-targets (Software from Dr. Feng Zhang's Lab, http://crispr.mit.edu). The complex crRNA:tracrRNA was mixed at equimolar ratio in the Nuclease-Free Duplex IDT buffer, incubated at 95°C for 5 minutes and hybridized at room temperature for 20 additional minutes. The ribonucleoprotein was then formed by incubating 0.1 µM of the specific crRNA:tracrRNA complexes, 1U of Cas9 Nuclease 3NLS and 5.0 µl of Lipofectamine® RNAiMAX in 40 µl of OptiMEM final volume (Life Technologies, CA, USA). The mixtures were placed at room temperature for 20 minutes to allow ribonucleoprotein complex formation. After this incubation, complexes were supplemented with one ml of DMEM without antibiotics and deposited in a well of 6-well plates for inversed transfection. Then, 200 x 10³ cells were added to each well and incubated at 37°C for 48 hours.

The significant reduction of apoptotic cells after silencing of HERV-V1/V2 genes shown in **Figure 7A** plausibly demonstrates, that HDACi-induced HERV transcription serves as an inducer or amplifier of TLR7/8 mediated apoptosis that is dramatically enhanced by additional application of specific agonists of TLR7/8.

### EXAMPLE 8

### Peripheral Blood Mononuclear Cells (PBMC) from healthy volunters as negative control: lacking induction of HERV-transcription by HDACi as well as missing synergistic effects of HDACi and agonists of TLR7/8 on apoptosis

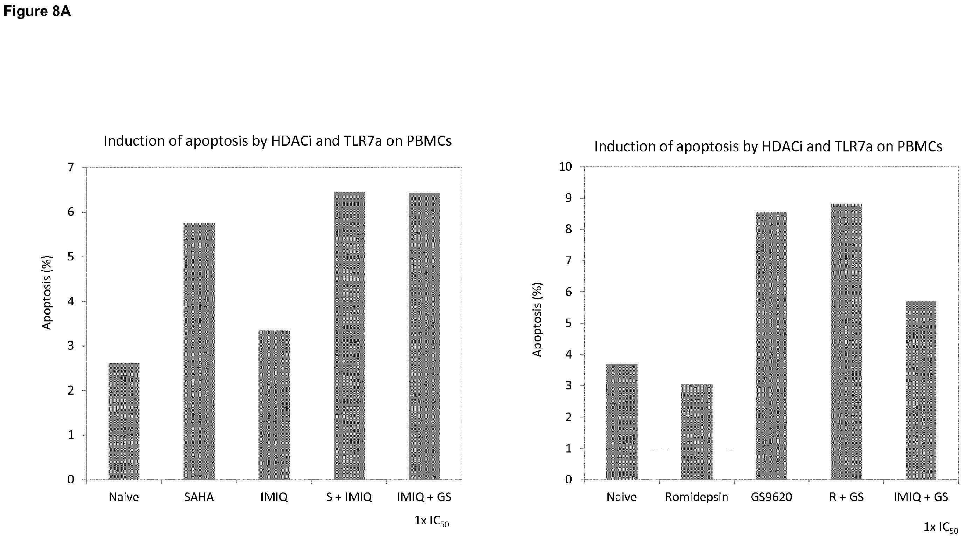

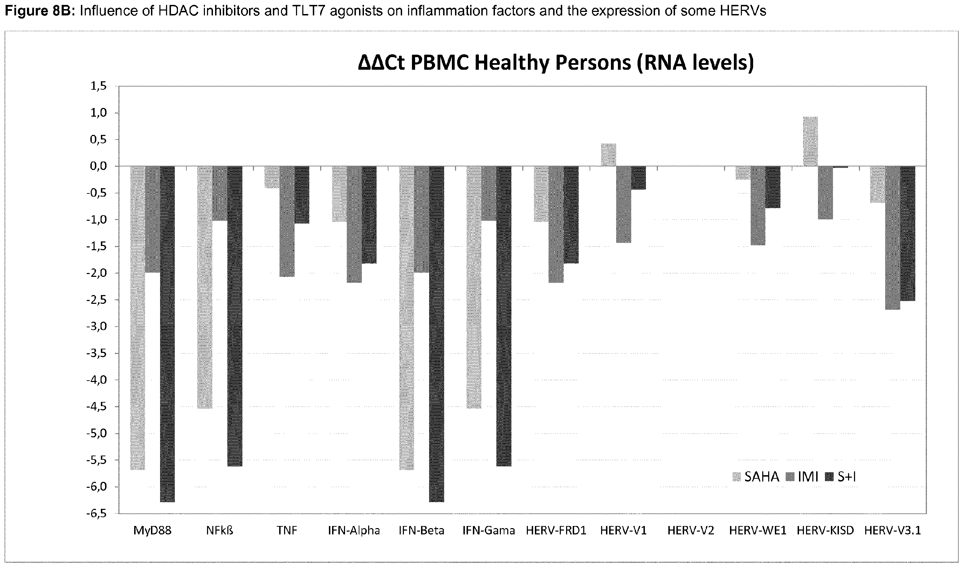

Regular, non-malignant Peripheral Blood Mononuclear Cells (PBMC) from healthy volunteers served as negative control in the following experiments **(****Figure 8A** and **8B****).**

Studies on the induction of apoptosis after the simultaneous incubation with HDACi and TLR7/8 agonists were carried out in **Figure 8A****..** HDACis alone, i.e. Vorinostat (SAHA) and Romidepsin, induced a rate of apoptosis of about 6% and 3%, respectively. In addition, TLR7/8 agonists alone, i.e. Imiquimod showed about 3% and Vesatolimod (GS9620) about 8% of apoptotis cells after 24h of drug exposure (1x IC50 in all experiments). The combination of both HDACi and TLR7/8 agonists did not reveal more apoptosis events as the single drugs, demonstrating that this combination did not induce a synergistic interaction as compared to cancer cells. The drug doses (1x IC50) employed for both compound classes were those obtained in SKOV3 cells. No gender differences were observed in a pilot of 10 persons.

Another relevant result is the fact that we found in healthy PBMCs of more than 20 donors that HERV-V1 and HERV-V2 were not overexpressed after HDACi exposure (**Figure 8B**).

The above results demonstrate that the combination of both HDACi and TLR7/8 agonists did not reveal more apoptosis events as the individual drugs alone, demonstrating that this combination did not induce a synergistic interaction as observed in cancer cells.

### EXAMPLE 9

### HDAC inhibitors induce the transcription of HERV-elements in a dose-dependent fashion

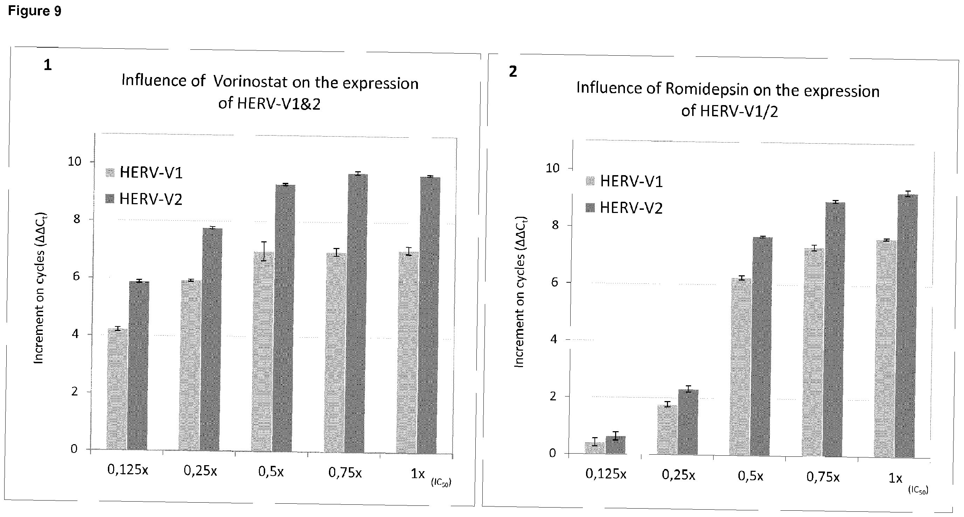

HDAC inhibitors enhanced the transcription of HERV-elements in SKOV3 cells. In particular, the expression of HERV-V1 and V2 was increased by incubation with HDACis, e.g. SAHA (Vorinostat) or Romidepsin, as shown in **Figure 9****.** It appears that HERV-V2 which encodes the full length envelope protein, was more activatable than its truncated form V1. Furthermore, the results clearly show enhanced activation of transcription by the HDACi Vorinostat (SAHA) at 1x IC50 compared to 0,5 x IC50 reflected by a dose-dependent increment of cycles.

Quantification of the amplification of HERV-V2/V1 transcription by Vorinostat via quatitative PCR (qPCR) is performed using 18S ribosomal RNA as standard (see also **Figure 1B****)..** The term ΔCt reflects the difference (Δ) in cycle (C) thresholds (t). For standardized qPCR, the reference is usually the house-keeping gene 18S ribosomal RNA, which is always expressed in all cells in comparable quantity. Measurement of specific RNA transcripts like HERV-V1/V2 by qPCR is usually performed in relation to the standard 18S, more or less transcription products are expressed in + or - ΔCt. Furthermore, differences in ΔCt between treated and untreated cells were shown as ΔΔCt.

The results presented in this example demonstrate that HDAC inhibitors induce the transcription of HERV-V1 and HERV-V2 in a dose-dependent fashion.

### EXAMPLE 10

### In-vivo confirmation of synergistic anti-cancer activity of HDACi (Romidepsin) in combination with an agonist of TLR7 (Vesatolimod) in a patient-derived tumor ectopic xenograft ovarian carcinoma mouse model

**Figure 10** presents confirmatory data in a patient-derived tumor ectopic xenograft ovarian carcinoma mouse model.

### Disseminated SKOV3^{WT} xenograft model

A total of 1x10⁶ of SKOV3^{WT} ovarian carcinoma cells were i.v. injected into six to eight week old female NMRI nude mice, via the tail vein. Romidepsin and Vesatolimod were intraperitoneally administrated at doses of 1 mg/kg and 5 mg/kg body weight four times at 3 day intervals for 12 consecutive days. These doses were considered as sub-toxic compared to doses applied in reports. At day 14, animals were narcotized in an isoflurane atmosphere and sacrificed by cervical dislocation. Lungs were extracted and fixed by immersion in 4% formaldehyde overnight and then stored in 70% ethanol prior to paraffin embedding. H & E staining was performed as previously described.

### Subcutan SKOV3^{WT} xenograft model

1 mm³ tumor pieces were implanted subcutaneosly into six to eight week old female NMRI nude mice by minimal operative intervention. Mice were randomized into treatment groups of 4. Three days after mice were were intraperitoneally treated at doses of 1 mg/kg and 5 mg/kg body weight four times at 3 day intervals for 12 consecutive days. Volumen of tumors were calculated by the formula *V*= 0.5236(LWT), using the values from the width (W), length (L) and thickness (T).

EDTA-blood was used for biochemical analysis for monitoring renal and hepar functions, as well as hematopoiesis. In **Figure 10A** we could clearly show that each drug alone did reduce the total tumor volume, the HDACi Romidepsin in the used dose more than the TLR7 agonist Vesatolimod. However, the drug combination did synergistically reduce the tumor volume without providing additional toxicity to the animals. These effects were also reflected by tissue sections from the lung of these animals. The drug combination was associated with a major reduction of lung metastases compared to each single drug application **(****Figure 10B****)**

### EXAMPLE 11

### Effects of HDACis and TLR7/8 agonists on inflammatory factors on RNA-level

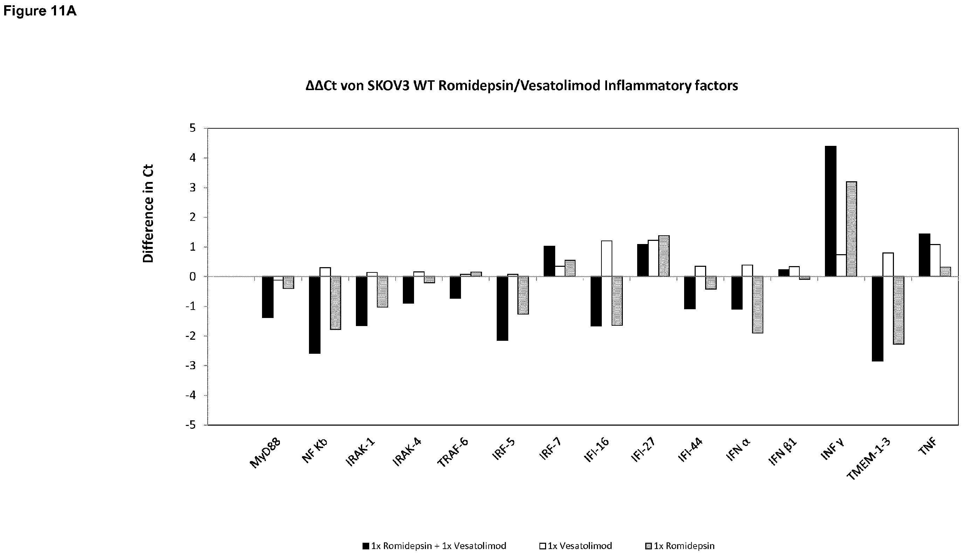

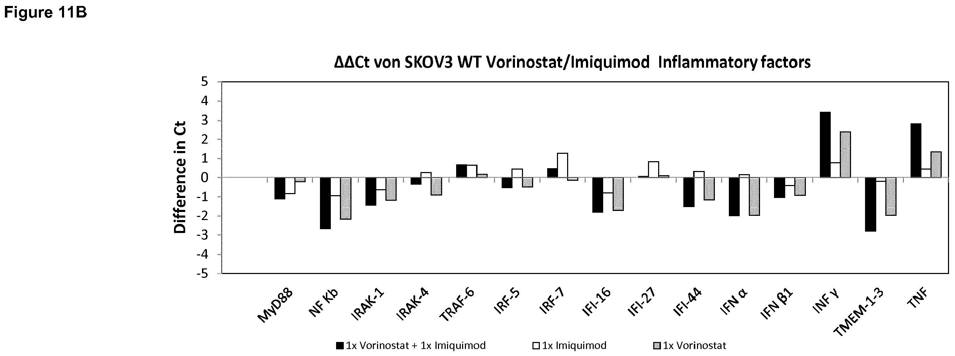

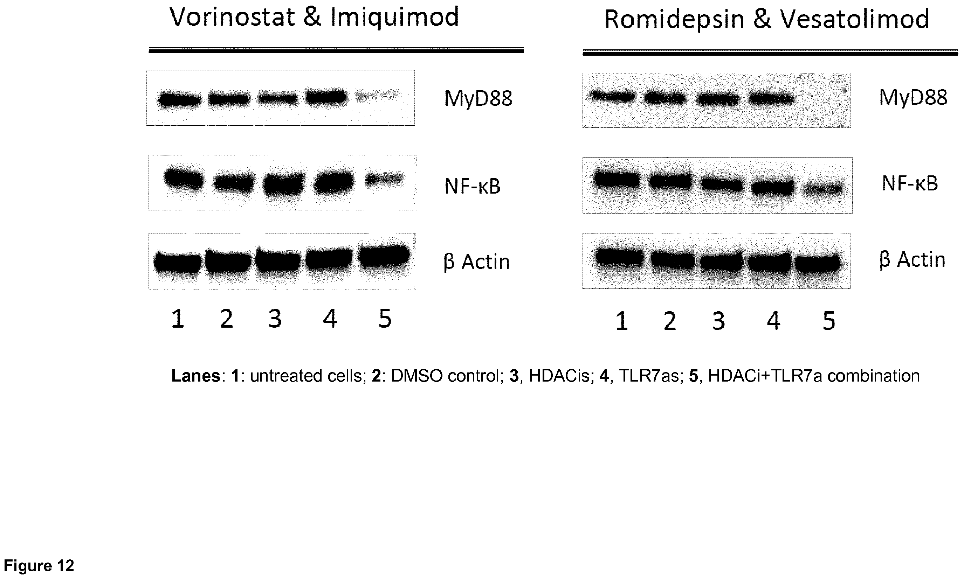

Since TLR agonists in general stimulate induction of the inflammasome, we examined the impact of the combination of HDACi and TLR7/8 agonists on inflammatory signaling. As depicted in **Figure 11****,** no major effects were observed. Only IFN Gamma was found to be upregulated. Importantly, the signaling via MyD88 was deactivated by its down-regulation at both RNA (**Figure 11**) and protein levels (**Figure 12**).

Quantification of the potential amplification of transcription of inflammatory factors by Romidesin/Vesatolimod or Vorinostat/Imiquimod via quatitative PCR (qPCR) is performed using 18S ribosomal RNA as standard (see also **Figure 1B****).**. The term ΔCt reflects the difference (Δ) in cycle (C) thresholds (t). For standardized qPCR, the reference is usually the house-keeping gene 18S ribosomal RNA, which is always expressed in all cells in comparable quantity. Measurement of specific RNA transcripts by qPCR is usually performed in relation to the standard 18S, more or less transcription products are expressed in + or - ΔCt. Furthermore, differences in ΔCt between treated and untreated cells were shown as ΔΔCt.

**Figure 12** shows a Western blot analysis revealing the downregulation of TLR7/8 signaling elements. Experiments were performed in SKOV3 ovarian carcinoma cells. Lanes 1 untreated, 2 vehicle control, 3 HDACis, 4 TLR7/8 agonists and 5 both drugs. Both, MyD88 and NF-Kβ were found under-regulated after the simultaneous exposure to both HDACi and TLR7 agonists. The doses employed were the respective IC₅₀-values for this cell line.

It is well reported, that after binding ligands, TLRs change structure for the recruitment of myleloid differentiation primary-response protein 88 (MYD88). MYD88 was also reported as a key regulator of the antiproliferative effect of HDAC inhibition. Downstream effects of the My88-dependent signaling pathway activate transcription factors like NF-κB and interferon regulatory factors (IRFs) that regulate inflammatory response and tumor dynamics
On the protein level, we found no interaction of either HDACi or TLR7 agonist with either MYD88 or NF-kB protein expression. However, the combination of HDACi plus TLR7 agonist resulted in dramatic loss of MYD88 protein level, which was also detectable, although to a lesser extent, for NF-kB. This observation was independent of the specific HDACi or TLR7 agonst used (**Figure 12****).** This finding was also confirmed in primary cancer cells isolated from patients. This loss of protein level regarding MYD88 and KF-kB was associated with a significant loss of RNA as measured by qPCR **(****Figure 11**), so that downregulation takes place most likely on transcriptional level.

Taken together, our data suggest an induction of apoptosis by the combination HDACi + TLR7 agonist not being dependent on the TLR adaptor protein MYD88, since protein expression seems completely downregulated on this drug combination.

### EXAMPLE 12

### The combination of HDACis and TLR7/8 agonists impaired signal transduction pathways linked to cell survival / cell growth and/or to cell proliferation

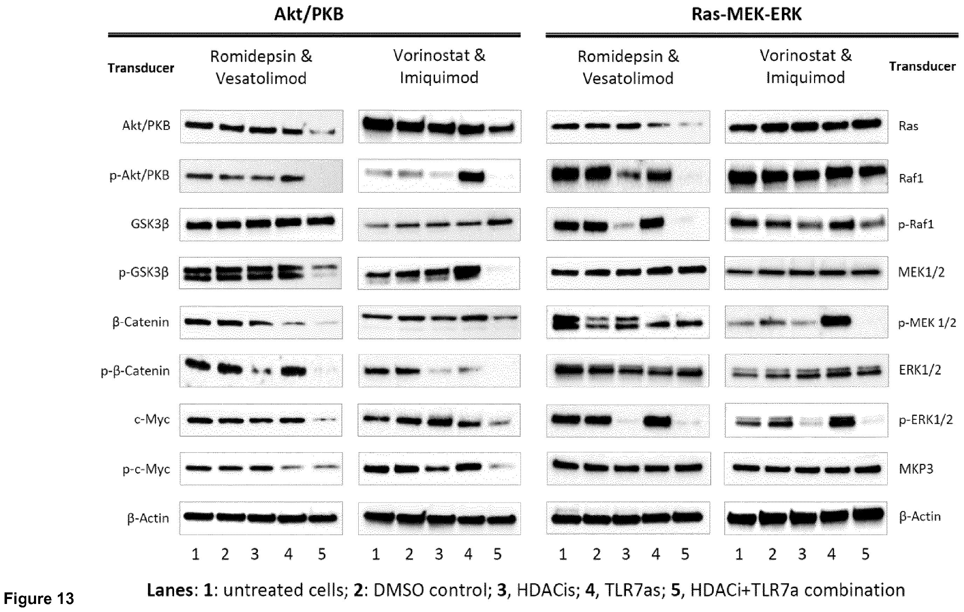

Major signal transduction pathways were found to be impaired by the combined exposure to HDACi and TLR7/8 agonist, as can be seen in **Figure 13****.**

Akt/PKB and Ras-MEK-ERK pathways were found only interrupted after the simultaneous incubation of HDACi and TLR7/8 agonists as studied by Western blots. Lane 1 untreated, lane 2 vehicle control, lane 3 HDACis, lane 4 TLR7/8 agonists and lane 5 both drugs. Both, MyD88 and NF-Kβ were found downregulated (**Figure 11****)** after the simultaneous exposure to both HDACi and TLR7 agonists. The doses employed were the respective IC₅₀-values for this cell line.

These results further explain the mechanism of action which connects TLR7/8 activation and downstream intrinsic apoptosis. It was found that major signal transduction cascades like Ras-ERK and Akt/PKB-Beta Catenin were interrupted. In cancer cells, MyD88-adaptor constitutively impedes the activity of MKP3 and with this the de-phosphorylation of MEK1/2 and ERK1/2 transducers, amplifying the activation of those transducers. Thus, the induced downregulation of MyD88, as shown in **Figure 12****,** indirectly interrupts this cascade which is of paramount importance in cell proliferation. Moreover, some HERV elements have been associated with the activation of signal cascades, in particular with the ERK1/2. Thus, their interruption via degradation of HERV elements may lead to apoptosis, inhibiting migration and invasion. Regarding Akt/PKB-Beta-Catenin interruption by the combination of HDACi and TLR7/8 agonists, it becomes clear that this induces a downregulation of c-Myc protein levels. This transcription factor governs a large number of genes involved in cell proliferation as well as the activation of apoptosis-related effectors. Thus, its impairment leads to inhibition of cell proliferation and finally to apoptosis.

### EXAMPLE 13

### Synergistic effects of HDACi and agonists of TLR7 and/or TLR8 also in the breast cancer cell line MCF7

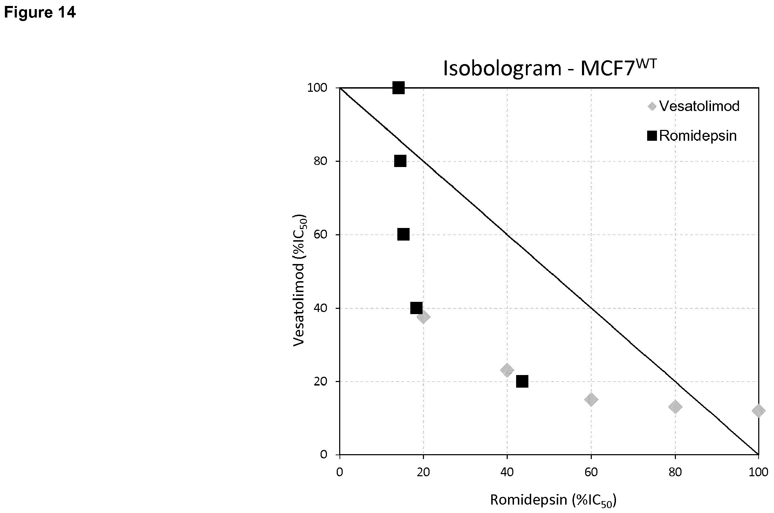

We want to point out that all effects previously reported for ovarian cancer (SKOC3 cell line, primary ovarian cancer cells from heavily treated patients) are not limited to this particular cancer type. We found comparable results for other tumor types like breast cancer, lung cancer and pancreatic cancer. As an example for another tumor type, we present data for the breast cancer cell line MCF7 that was incubated with a combination of HDACi (Romidepsin) and TLR7/8 agonist (Vesatolimod = GS9620). As demonstrated in the isobologram shown in **Figure 14****,** the combination of Romidepsin and Vesatolimod shows also a synergistic effect on the proliferation in the breast cancer cell line MCF7.

The endpoint used in **Figure 14** is inhibition of proliferation by 50% (IC₅₀) (**see also** **Figure 3****).** Specifically, all dots in **Figure 14** reflect a combination of HDACi plus TLR7/8 agonist, which resulted in 50% growth inhibition. All dots on the diagonal reflect additive activity (or lacking interaction), for instance 100% IC₅₀ of drug A und 0% IC₅₀ of drug B or vice versa. Dots above the diagonal would reflect antagonistic interactions, which was not a result of this experiment. However, all dots in experiments carried out for the results shown in **Figure 14** below the diagonal strongly suggest synergistic antiproliferative interactions between the analyzed HDACi Romidepsin and the TLR7 agonist Vesatolimod.

The present study aimed to develop an efficacious therapy for treating cancer using a combination of at least one HDACi and at least one agonist of TLR7 and/or TLR8.

As the above results clearly indicate the combination of at least one HDACi and at least one agonist of TLR7 and/or TLR8 in the treatment of cancer allowed a significant improvement in the treatment of cancer suggesting that the treatment could be extended to patients having chemo-resistant cancer who would otherwise remain resistant to treatment with cytotoxic drugs. Such combination treatment can be in the form of a combined administration of the at least one HDACi and at least one agonist of TLR7 and/or TLR8, wherein these two classes of compounds can be combined within a pharmaceutical composition. Such combination treatment of cancer has not been described in the prior art.

The invention represents a novel concept in the treatment of cancer with translational relevance. It is based on the simultaneous use of HDACi and TLR7/8 agonists, which in sub-toxic doses induce a synergistic cytotoxic effect via intrinsic apoptosis, without an activation of the inflammasome in cancer cells. This concept is based on the re-activation of some endogenous retroviral elements, such as HERV-V1 and HERV-V2, by HDACi and the induction of major apoptosis using agonists of TLR-7/8, which represent pattern recognition receptors of the innate immune system, involved in the recognition of viral signatures. Although, this approach was shown to be highly active in ovarian cancer cells and breast cancer cells, additional studies in pancreatic carcinoma cells as well as in lung cancer cells showed similar effects.

In conclusion, HDACi as inducers of endoretroviral transcripts in combination with agonists of TLR7/8 resulted in significant and clinically relevant apoptosis in cancer cells, such as ovarian cancer cells, but not in normal, healthy cells. This effect is not restricted to a particular drug used. Exclusion of major inflammatory factors makes this novel therapeutic strategy attractive as a novel option for treating chemo-resistant ovarian cancer as well as other chemo-resistant cancer types.

## Claims

1. A pharmaceutical composition comprising at least one inhibitor of a histon-deacetylase (HDACi) and at least one agonist of Toll-like-receptor 7 and/or 8 (TLR7 and/or TLR8).

2. The pharmaceutical composition of claim 1, wherein the inhibitor of a histon-deacetylase (HDACi) is selected from one or more of the group consisting of vorinostat (SAHA), romidepsin and panobinostat.

3. The pharmaceutical composition of claim 1 or claim 2, wherein the agonist of Toll-like-receptor 7 and/or 8 (TLR7 and/or TLR8) is selected from one or more of the group consisting of vesatolimod (GS9620), imiquimod and resiquimod (R 848).

4. The pharmaceutical composition of claims 1-3, wherein the HDACi is romidepsin and the agonist of TLR7 and/or TLR8 is vesatolimod, or, wherein the HDACi is vorinostat and the agonist of TLR7 and/or TLR8 is imiquimod.

5. The pharmaceutical composition of claims 1-4 further comprising a pharmaceutically acceptable excipient.

6. The pharmaceutical composition of claims 1-5 for use in a method of treating or preventing a disease.

7. The pharmaceutical composition of claims 1-5 for use in a method of treating or preventing cancer.

8. The pharmaceutical composition for use according to claim 7, wherein the cancer is based on a non-hematological malignancy, wherein the cancer is preferably selected from the group consisting of ovarian cancer, breast cancer, pancreatic cancer and lung cancer.

9. The pharmaceutical composition for use according to claims 7-8, wherein the cancer is chemoresistant cancer, wherein the chemoresistant cancer is preferably resistant to cytotoxic drugs.

10. The pharmaceutical composition for use according to claims 7-9, wherein the cancer is ovarian cancer, preferably chemo-resistant ovarian cancer, and more preferably chemo-resistant ovarian cancer that is resistant to cytotoxic drugs.

11. The pharmaceutical composition for use according to claims 6-10, wherein the pharmaceutical composition is administered parenterally, such as intravenously, or wherein the pharmaceutical composition is administered orally.

12. A cancer cell having been contacted with the pharmaceutical composition of claims 1-5, wherein said cancer cell has an upregulated expression of one or more single-stranded RNA (ssRNA) genes, wherein the one or more ssRNA genes with upregulated expression are preferably endogenous retroviral (HERV) genes.

13. The cancer cell of claim 12, wherein the one or more HERV genes with upregulated expression comprise HERV-V1 and/or HERV-V2, wherein the cancer cell preferably is an isolated cancer cell.

14. An *in vitro* method for contacting cancer cells with the pharmaceutical composition of claims 1-5 comprising the step of:
a) contacting cancer cells isolated from a cancer patient with the pharmaceutical composition of claims 1-5.

15. A population of cancer cells obtainable by the method of claim 14.


SEQUENCE LISTING

<110>  Prof. Dr. Dirk Strumberg

<120>  Pharmaceutical compositions comprising an inhibitor of a
       histon-deacetylase (HDACi) and an agonist of Toll-like-receptor 7
        and/or 8 (TLR7 and/or TLR8) and their use in the treatment of
       cancer

<130>  EP124789-FZ358pau

<140>  not yet assigned
<141>  herewith

<160>  2

<170>  PatentIn version 3.5

<210>  1
<211>  23
<212>  DNA
<213>  Artificial Sequence

<220>
<223>  gRNA

<400>  1
tatatatcat aaagaaaact cgg                                              23


<210>  2
<211>  23
<212>  DNA
<213>  Artificial Sequence

<220>
<223>  gRNA

<400>  2
gatcagccca tatggacaac agg                                              23